(19) Europäisches Patentamt
European Patent Office
Office européen des brevets

(11) **EP 4 643 783 A1**

(12) **EUROPEAN PATENT APPLICATION**
published in accordance with Art. 153(4) EPC

(43) Date of publication:
05.11.2025 Bulletin 2025/45

(21) Application number: 23910652.9

(22) Date of filing: 26.12.2023

(51) International Patent Classification (IPC):
*A61B 6/03* (2006.01)    *G06T 11/00* (2006.01)
*A61B 6/00* (2024.01)    *A61B 5/055* (2006.01)

(52) Cooperative Patent Classification (CPC):
A61B 5/055; A61B 6/00; A61B 6/03; G06T 11/00

(86) International application number:
PCT/CN2023/142100

(87) International publication number:
WO 2024/140729 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.12.2022 CN 202211736267

(71) Applicant: RAYSOLUTION Healthcare Co., Ltd
Hefei, Anhui 230093 (CN)

(72) Inventors:
• FANG, Lei
Hefei, Anhui 230093 (CN)

• ZHANG, Bo
Hefei, Anhui 230093 (CN)
• CHEN, Weicao
Hefei, Anhui 230093 (CN)
• YANG, Lingli
Hefei, Anhui 230093 (CN)
• HU, Yun
Hefei, Anhui 230093 (CN)

(74) Representative: Müller Verweyen
Patentanwälte
Friedensallee 290
22763 Hamburg (DE)

(54) **TIME CORRECTION METHOD AND APPARATUS, DEVICE, AND COMPUTER-READABLE STORAGE MEDIUM**

(57) In the disclosure disclosed are a time calibration method, device, apparatus, and computer-readable storage medium. The time calibration method comprises determining lines of response passing through a target object and coincidence events on each line of response based on sampling data of the target object, acquiring an activity image, and determining pixel points where the respective lines of response passing through the target object intersect with the activity image, and determining a pixel value-time difference statistical distribution corresponding to each line of response based on the pixel points respectively; determining a coincidence event-time difference statistical distribution corresponding to each line of response based on the coincidence events on each line of response; calculating a maximum value of inner product of the pixel value-time difference statistical distribution and the coincidence event-time difference statistical distribution and determining a time offset difference value corresponding to the maximum value $Y_i$ for each line of response; and determining a time calibration value based on the offset difference value. The disclosure is widely applicable, does not require prolonged sampling, thereby saving time and enhancing the efficiency of time calibration.

EP 4 643 783 A1

Based on the sampling data of the target object, determining lines of response LORi (i    passing through the target object and coincidence events Ki (i    on each LORi

S120

Based on all coincidence events of all lines of response LORi passing through the target object, acquiring an activity image, and determining pixel points Pi where each line of response LORi passing through the target object intersects with the activity image, and determining a pixel value-time difference statistical distribution Ti corresponding to each LORi based on the pixel points Pi respectively

S130

Based on the coincidence events Ki on each line of response LORi, determining a coincidence event-time difference statistical distribution Mi corresponding to each line of response LORi

S140

Based on the pixel value-time difference statistical distribution Ti and the coincidence event-time difference statistical distribution Mi corresponding to each LORi, calculating the maximum value Yi of the inner product of each Ti and Mi, and determining a difference value $\delta\triangle i$ of the time offsets corresponding to the maximum value for each LORi

S150

Determining a time calibration value based on the $\delta\triangle i$

S160

FIG. 2

**Description**

The present application claims the priority of Chinese Patent Application No. 202211736267.8, filed on December 30, 2022, filed on, the entire content of which is incorporated by reference.

**Technical Field**

This disclosure relates to the field of data processing, and particularly relates to a time calibration method, device, apparatus and computer-readable storage medium.

**Background**

Positron Emission Tomography (abbreviated as PET) technology is currently one of the most advanced molecular imaging technologies globally. Through imaging compounds labeled with radioactive nuclides within biological bodies, it can non-invasively, quantitatively and dynamically evaluate metabolic levels, biochemical reactions and functional activities of various functional organs within biological bodies, having high sensitivity and accuracy.

In order to obtain the distribution of positron radioactive nuclides in human body, the detection principle of PET system is that positron nuclide decay produces a positron, the positron annihilates with surrounding electrons, producing a pair of gamma photons with opposite directions and energy of 511 keV each, and if two gamma photons are respectively detected by two scintillation crystal bars of the PET detector, then the line connecting the center surfaces of these two scintillator bars is called a line of response (abbreviated as LOR). If two scintillators located on an LOR in the detector respectively detect two gamma photons within a specified coincidence time window (for example, 0~15 nanoseconds), that is, the difference value of time information of two gamma photons falls within the coincidence time window, and the energy of both gamma photons falls within the coincidence energy window, then the event detecting these two gamma photons may be called a coincidence event, and the event detecting any one gamma photon is called a single event.

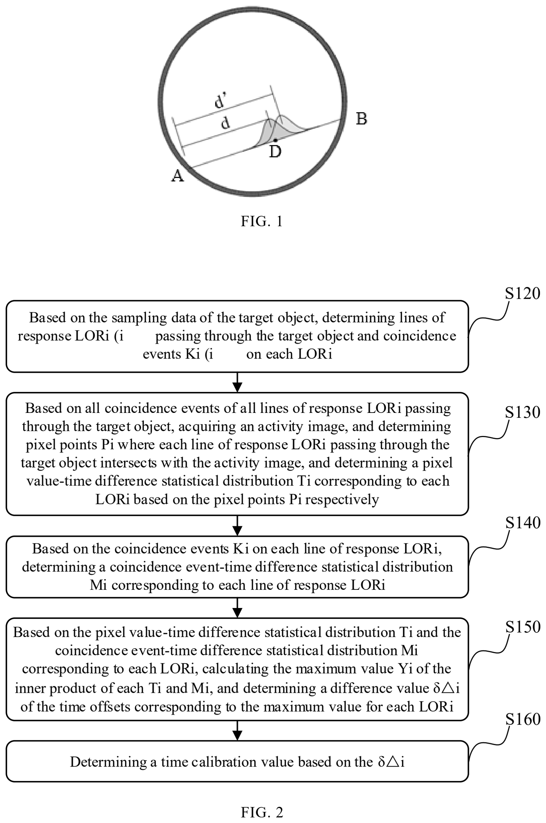

As shown in Figure 1, assuming two gamma photons produced by an annihilation event are respectively detected at point A (scintillator bar A) and point B (scintillator bar B) of the PET detector, then the connecting line of AB is called a line of response (LOR).

Theoretically, if the times tA and tB when scintillation crystal bars A and B of the detector detect gamma photons are sufficiently precise, then the position D where the annihilation occurs may be precisely determined. Let the length of AB be L, the distance from point D to point A be d, the speed of light be C, then d = L/2 + C/2*(tA-tB). That is, through the precisely collected arrival times of gamma photons, the position of nuclide annihilation may be directly calculated, without the need of complex image reconstruction method to obtain PET image.

However, in actual systems, due to differences in detector scintillator bar materials, differences in optical path lengths, differences in electronic timing, there will be background delay for the scintillator bars, that is, time difference, leading to the actual detection times at points A and B to be tA' and tB', tA'= tA+$\delta$A, tB'= tB+$\delta$B. Then $\delta$A and $\delta$B are respectively the time offsets of scintillator bar A and scintillator bar B. In view of the existence of time offset, the distance from A to the radioactive nuclide annihilation position calculated based on tA' and tB' is d'=L/2+C/2*(tA'-tB') = d+C/2*($\delta$A-$\delta$B), d = d'-C/2*($\delta$A-$\delta$B) = L/2 + C/2*(tA'-tB') - C/2*($\delta$A-$\delta$B), that is, the acquired radioactive nuclide annihilation position is offset from the actual annihilation position D.

In order to acquire accurate annihilation position, it is needed to perform time calibration on the time data detected by scintillators.

Typically, it acquires time offsets $\delta$1~$\delta$n between the actual measured time values and theoretical gamma photon arrival times for all n scintillation crystal bars of the detector, and uses these offset values to calibrate the actually acquired time information, which process is called time offset calibration, i.e., time calibration of scintillators, and therefore the critically important work in the time calibration process is to obtain time offsets $\delta$1~$\delta$n between actual measured time values and theoretical gamma photon arrival times for all scintillator bars.

In prior art time calibration methods, in order to obtain time offsets $\delta$1~$\delta$n between actual measured time values and theoretical gamma photon arrival times for scintillator bars, it is needed to characterize the time offsets of scintillation crystal bars at both ends of the LOR. Currently, typically based on the time differences of coincidence events corresponding to an LOR to characterize the time offsets of scintillation crystal bars at both ends of that LOR. However, theoretically, for gamma rays emitted from different target objects, when characterizing the difference in time offsets of scintillation crystal bars at both ends of an LOR, the methods for obtaining mean time difference of coincidence events corresponding to the LOR are different, and various methods cannot be universally used for different target objects. For example: for the case of using a certain uniform-shaped target objects (such as small cylinder or line source), the mean sum of time differences of all coincidence events on that LOR may be used as the mean time difference of coincidence events corresponding to the LOR; for the case of currently ring-shaped target objects (such as target objects of shell source or shell source-like target objects formed by rotating a line source a revolution), when the activity and shape of target object are both uniform, the mean sum of time differences of all coincidence events on the LOR may be used as the mean time difference of coincidence events corresponding to that LOR; when target object's activity and shape are not uniform, the mean sum of time differences of all coincidence events on the LOR cannot be used as the mean time difference of coincidence events corresponding to that LOR, but it may usually, through Gaussian fitting, acquire the mean sum of respective time differences corresponding to each annihilation position as the mean time difference of coincidence events corresponding to that LOR; Gaussian fitting method has to collect enough counts from the target object, with a very long acquisition time, at least 1h, consuming too much time; or requires very high drug activity, but high drug activity will cause more random events, increasing measurement errors.

In summary, in current time calibration methods, in order to obtain time offsets $\delta_1 \sim \delta_n$ between actual measured time values and theoretical gamma photon arrival times for scintillator bars, when characterizing the difference in time offsets of scintillation crystal bars at both ends of the LOR, the following deficiencies exist: for gamma rays produced from different target objects, the methods for characterizing the difference in time offsets of scintillator bars at both ends of the LOR are different, and various methods cannot be universally used, there is no standardized approach for characterizing the difference value of time offsets of scintillator bars at both ends of that LOR, and some methods are time-consuming and produce results with significant errors.

The background description is provided merely to aid in understanding of the background of the present disclosure, and does not constitute an admission of prior art.

## Summary

The disclosure intends to provide a time calibration method, device, apparatus and computer-readable storage medium that can solve at least one problem existing in the prior art.

According to a first aspect of the disclosure, a time calibration method is provided, comprising: based on sampling data of a target object, determining lines of response (LORi) passing through the target object and coincidence events Ki on each LORi, wherein i represents a number, i≥1; acquiring an activity image based on all coincidence events of all lines of response LORi passing through the target object, and determining pixel points Pi where the respective LORi passing through the target object intersect with the activity image, and determining a pixel value-time difference statistical distribution Ti corresponding to each LORi based on the pixel points Pi respectively; determining a coincidence event-time difference statistical distribution Mi corresponding to each LORi based on the coincidence events Ki on each line of response LORi; based on the pixel value-time difference statistical distribution Ti and the coincidence event-time difference statistical distribution Mi, calculating a maximum value Yi of inner product of the pixel value-time difference statistical distribution Ti and the coincidence event-time difference statistical distribution Mi, and determining a time offset difference value $\delta\triangle i$ corresponding to the maximum value Yi for each line of response LORi; and determining a time calibration value based on the offset difference value $\delta\triangle i$.

According to an embodiment of the disclosure, the lines of response LORi passing through the target object comprise: the lines of response LORi having at least one intersection point with the target object, or the lines of response LORi being externally tangent to or intersecting with the target object.

According to an embodiment of the disclosure, based on sampling data of a target object, determining lines of response (LORi) passing through the target object and coincidence events Ki on each LORi, comprises: determining all coincidence events and lines of response LORs corresponding to each coincidence event based on the sampling data; and screening all lines of response LORi passing through the target object and coincidence events Ki on each LORi.

According to an embodiment of the disclosure, screening all lines of response LORi passing through the target object and coincidence events Ki on each LORi, comprises: determining whether a straight line where the line of response LOR lies has an intersection point with the target object based on priori information.

According to an embodiment of the disclosure, the priori information comprises the number of scintillator IPs between the spaced scintillators at two ends of the response line LOR, wherein the number of scintillator IPs between the spaced scintillators is determined based on whether the inner diameters of the target object in various directions intersect with the straight line where the response line LOR lies.

According to an embodiment of the disclosure, acquiring the activity image based on all coincidence events of all LORi passing through the target object, comprises: reconstructing the activity image using filtered back projection or an iterative method based on all coincidence events on all lines of response LORi passing through the target object.

According to an embodiment of the disclosure, determining pixel points Pi where the respective LORi passing through the target object intersect with the activity image, comprises: determining pixel points Pi where the respective, screened lines of response LORi intersect with the activity image based on a ray tracing method.

According to an embodiment of the disclosure, determining the pixel value-time difference statistical distribution Ti corresponding to each LORi based on the pixel points Pi respectively, comprises: calculating a time difference from each pixel point Pi to the endpoints of the corresponding line of response LORi for each pixel point Pi, and recording the pixel value of each pixel point Pi; determining a plurality of equally spaced time difference intervals, and accumulating pixel values of the pixel points Pi whose time differences fall within the respective time difference interval based on the time difference for each pixel point Pi; and determining the pixel value-time difference statistical distribution Ti corresponding to each line of response LORi by taking the time difference interval as a horizontal axis step size and taking the accumulated pixel value of the pixel points falling within each step size as a vertical axis.

According to an embodiment of the disclosure, determining a coincidence event-time difference statistical distribution Mi corresponding to each LORi based on the coincidence events Ki on each line of response LORi, comprises: determining a horizontal axis by taking the horizontal axis step size of the pixel value-time difference statistical distribution Ti as a step size, and by taking the accumulated value of coincidence events whose time differences fall within the respective step size as a vertical axis, determining the coincidence event-time difference statistical distribution Mi corresponding to each line of response LORi.

According to an embodiment of the disclosure, calculating the maximum value Yi of inner product of the pixel value-time difference statistical distribution Ti and the coincidence event-time difference statistical distribution Mi and determining the time offset difference value $\delta\triangle i$ corresponding to the maximum value Yi for each line of response LORi, comprises: using the pixel value-time difference statistical distribution Ti as a reference, shifting the coincidence event-time difference statistical distribution Mi, calculating the inner product of the pixel value-time difference statistical distribution Ti and the coincidence event-time difference statistical distribution Mi, and statistically acquiring a shift amount of the coincidence event-time difference statistical distribution Mi when the maximum value Yi of the inner product is reached, the shift amount being the offset difference value $\delta\triangle i$.

According to an embodiment of the disclosure, determining a time calibration value based on the offset difference value $\delta\triangle i$, comprises: based on the difference value $\delta\triangle i$ of the time offsets, determining the relationship between the time offsets $\delta ci$ and $\delta di$ of the scintillators ci and di at two ends of each response line LORi, where $\delta ci - \delta di = \delta\triangle i$; and determining the time offsets $\delta ci$ and $\delta di$ of the scintillators ci and di at two ends of each line of response LORi by solving a system of equations using a fitting method, the time offsets $\delta ci$ and $\delta di$ being the time calibration values of the scintillators ci and di.

According to an embodiment of the disclosure, the time calibration method further comprises: performing time calibration on the sampling data based on the determined time calibration values, that is, performing calibration on time data corresponding to the scintillators ci and di in the sampling data based on the time offsets $\delta ci$ and $\delta di$ of scintillators ci and di.

According to an embodiment of the disclosure, performing time calibration on the sampling data based on the determined time calibration values, comprises: subtracting the time calibration values $\delta ci$ and $\delta di$ from the time data tci and tdi corresponding to the scintillators ci and di in the sampling data, respectively.

According to a second aspect of the disclosure, a time calibration method is provided, comprising: based on sampling data of a target object, determining lines of response LORi passing through the target object and coincidence events Ki on each LORi, wherein i represents a number, i≥1; acquiring an activity image based on all coincidence events of all lines of response LORi passing through the target object; based on all lines of response LORi passing through the target object, screening lines of response LORj on which coincidence events' number satisfies a preset condition, wherein j represents a number, j≥1; determining pixel points Pj where the respective, screened lines of response LORj intersect with the activity image, and determining a pixel value-time difference statistical distribution Tj corresponding to each line of response LORj based on the pixel points Pj respectively; determining a coincidence event-time difference statistical distribution Mj corresponding to each screened line of response LORj; based on the pixel value-time difference statistical distribution Tj and the coincidence event-time difference statistical distribution Mj corresponding to each screened line of response LORj, calculating a maximum value Yj of the inner product of the pixel value-time difference statistical distribution Tj and the coincidence event-time difference statistical distribution Mj, and determining a time offset difference value $\delta\triangle j$ corresponding to the maximum value Yj for each line of response LORj; and determining a time calibration value based on the offset difference value $\delta\triangle j$.

According to an embodiment of the disclosure, the lines of response LORi passing through the target object comprise: the lines of response LORi having at least one intersection point with the target object, or the lines of response LORi being externally tangent to or intersecting with the target object.

According to an embodiment of the disclosure, based on sampling data, determining lines of response LORi passing through the target object and coincidence events Ki on each LORi, comprises: determining all coincidence events and lines of response LOR corresponding to each coincidence event based on the sampling data; and screening all lines of response LORi passing through the target object and coincidence events Ki on each LORi.

According to an embodiment of the disclosure, screening all lines of response LORi passing through the target object and coincidence events Ki on each LORi, comprises: determining whether a straight line where the line of response LOR lies has an intersection point with the target object based on priori information.

According to an embodiment of the disclosure, the priori information comprises the number of scintillator IPs between the spaced scintillators at two ends of the response line LOR, wherein the number of scintillator IPs between the spaced scintillators is determined based on whether the inner diameters of the target object in various directions intersect with the straight line where the response line LOR lies.

According to an embodiment of the disclosure, acquiring the activity image based on all coincidence events of all LORi passing through the target object, comprises: reconstructing the activity image using filtered back projection or an iterative method based on all coincidence events on all lines of response LORi passing through the target object.

According to an embodiment of the disclosure, based on all lines of response LORi passing through the target object, screening lines of response LORj on which coincidence events' number satisfies a preset condition, comprises: setting a reference value for comparing the number of coincidence events; determining whether the number of coincidence events is greater than the reference value; and storing lines of response LORj on which the coincidence events' number is greater than the reference value.

According to an embodiment of the disclosure, determining pixel points Pj where the respective, screened lines of response LORj intersect with the activity image, comprises: determining pixel points Pj where the respective lines of response LORj intersect with the activity image based on a ray tracing method.

According to an embodiment of the disclosure, determining a pixel value-time difference statistical distribution Tj corresponding to each line of response LORj based on the pixel points Pj respectively, comprises: calculating a time difference from each pixel point Pj to the endpoints of the corresponding line of response LORj for each pixel point Pj, and recording the pixel value of each pixel point Pj; determining a plurality of equally spaced time difference intervals, and accumulating pixel values of the pixel points Pj whose time differences fall within the respective time difference interval based on the time difference for each pixel point Pj; and determining the pixel value-time difference statistical distribution Tj corresponding to each line of response LORj by taking the time difference interval as a horizontal axis step size and taking the accumulated pixel value of the pixel points falling within each step size as a vertical axis.

According to an embodiment of the disclosure, determining the coincidence event-time difference statistical distribution Mj corresponding to each screened line of response LORj, comprises: determining a horizontal axis by taking the horizontal axis step size of the pixel value-time difference statistical distribution Tj as a step size, and by taking the accumulated value of coincidence events whose time differences fall within the respective step size as a vertical axis, determining the coincidence event-time difference statistical distribution Mj corresponding to each line of response LORj.

According to an embodiment of the disclosure, calculating a maximum value Yj of the inner product between the pixel value-time difference statistical distribution Tj and the coincidence event-time difference statistical distribution Mj, and determining a time offset difference value $\delta\triangle j$ corresponding to the maximum value for each line of response LORj, comprises: using the pixel value-time difference statistical distribution Tj as a reference, shifting the coincidence event-time difference statistical distribution Mj, calculating the inner product of the pixel value-time difference statistical distribution Tj and the coincidence event-time difference statistical distribution Mj, and statistically acquiring a shift amount of the coincidence event-time difference statistical distribution Mj when the maximum value Yj of the inner product is reached, the shift amount being the offset difference value $\delta\triangle j$.

According to an embodiment of the disclosure, determining a time calibration value based on the offset difference value $\delta\triangle j$, comprises: based on the difference value $\delta\triangle j$ of the time offsets, determining the relationship between the time offsets $\delta cj$ and $\delta dj$ of the scintillators cj and dj at two ends of each line of response LORj, where $\delta cj - \delta dj = \delta\triangle j$; and determining the time offsets $\delta cj$ and $\delta dj$ of the scintillators cj and dj at two ends of each line of response LORj by solving a system of equations using a fitting method, the time offsets $\delta cj$ and $\delta dj$ being the time calibration values of the scintillators cj and dj.

According to an embodiment of the disclosure, the time calibration method further comprises: performing time calibration on the sampling data based on the determined time calibration values, that is, performing calibration on time data corresponding to the scintillators cj and dj in the sampling data based on the time offsets $\delta cj$ and $\delta dj$ of scintillators cj and dj.

According to an embodiment of the disclosure, performing time calibration on the sampling data based on the determined time calibration values, comprises: subtracting the time calibration values $\delta cj$ and $\delta dj$ from the time data tcj and tdj corresponding to the scintillators cj and dj in the sampling data, respectively.

According to a third aspect of the disclosure, a time calibration device is provided, comprising: a coincidence event screening module, configured to based on sampling data of a target object, determine lines of response LORi passing through the target object and coincidence events Ki on each LORi, wherein i represents a number, $i \geq 1$; an activity image reconstruction module, configured to acquire an activity image based on all coincidence events of all lines of response LORi passing through the target object; a pixel value-time difference statistical distribution acquisition module, configured to determine pixel points Pi where the respective lines of response LORi passing through the target object intersect with the activity image, and determine a pixel value-time difference statistical distribution Ti corresponding to each LORi based on the pixel points Pi respectively; a coincidence event-time difference statistical distribution acquisition module, configured to determine a coincidence event-time difference statistical distribution Mi corresponding to each line of response LORi; a calculation module, configured to calculate a maximum value Yi of inner product of the pixel value-time difference statistical distribution Ti and the coincidence event-time difference statistical distribution Mi and determine a time offset difference value $\delta\triangle i$ corresponding to the maximum value Yi for each line of response LORi; and a time calibration value acquisition module, configured to determine a time calibration value based on the offset difference value $\delta\triangle i$.

According to an embodiment of the disclosure, the sampling data comprises scintillator IP information and time information of pulse signals; and the coincidence event screening module is further configured to based on the sampling data, determine energy information of the pulse signals, and determine coincidence events based on the time information and the energy information of the pulse signals, and determine the lines of response LOR corresponding to the coincidence events based on the scintillator IP information.

According to an embodiment of the disclosure, the time calibration device further comprises: a recording module, configured to record time differences of the coincidence events and scintillator IP information of scintillators at two ends of the corresponding line of response LOR.

According to an embodiment of the disclosure, the coincidence event screening module is further configured to based on priori information, determine whether a straight line where the line of response LOR lies has an intersection point with the target object, and determine whether the line of response LOR passes through the target object based on a number of intersection points.

According to an embodiment of the disclosure, the priori information comprises the number of scintillator IPs between the spaced scintillators at two ends of the response line LOR, wherein the number of scintillator IPs between the spaced scintillators is determined based on whether the inner diameters of the target object in various directions intersect with the straight line where the response line LOR lies.

According to an embodiment of the disclosure, the activity image reconstruction module is configured to reconstruct the activity image using filtered back projection or an iterative method.

According to an embodiment of the disclosure, the pixel value-time difference statistical distribution acquisition module is configured to determine pixel points Pi where the screened lines of response LORi intersect with the activity image based on a ray tracing method.

According to an embodiment of the disclosure, the pixel value-time difference statistical distribution acquisition module comprises: a pixel point time difference acquisition module, configured to calculate a time difference from each pixel point Pi to the endpoints of the corresponding line of response LORi, and record the pixel value of each pixel point Pi; a pixel value accumulation module, configured to determine a plurality of equally spaced time difference intervals, and accumulate pixel values of the pixel points Pi whose time differences fall within the respective time difference interval based on the time difference for each pixel point Pi; and the pixel value-time difference statistical distribution acquisition module is configured to determine the pixel value-time difference statistical distribution Ti corresponding to each line of response LORi by taking the time difference interval as a horizontal axis step size and taking the accumulated pixel value of the pixel points falling within each step size as a vertical axis.

According to an embodiment of the disclosure, the coincidence event-time difference statistical distribution acquisition module is configured to: determine a horizontal axis by taking the horizontal axis step size of the pixel value-time difference statistical distribution Ti as a step size, and by taking the accumulated value of coincidence events whose time differences fall within the respective step size as a vertical axis, determine the coincidence event-time difference statistical distribution Mi corresponding to each line of response LORi.

According to an embodiment of the disclosure, the time calibration value acquisition module is further configured to based on the difference value $\delta\triangle i$ of the time offsets, determine the relationship between the time offsets $\delta ci$ and $\delta di$ of the scintillators ci and di at two ends of each line of response LORi, where $\delta ci - \delta di = \delta\triangle i$; and determine the time offsets $\delta ci$ and $\delta di$ of the scintillators ci and di at two ends of each line of response LORi by solving a system of equations using a fitting method, the time offsets $\delta ci$ and $\delta di$ being the time calibration values of the scintillators ci and di.

According to an embodiment of the disclosure, the time calibration device further comprises a calibration module, configured to perform time calibration on the time information of pulse signals in the sampling data based on the determined time calibration values: subtracting the time calibration values $\delta ci$ and $\delta di$ from the time data tci and tdi corresponding to the scintillators ci and di in the sampling data, respectively.

According to a fourth aspect of the disclosure, a time calibration device is provided, comprising: a first coincidence event screening module, configured to based on sampling data of a target object, determine lines of response LORi passing through the target object and coincidence events Ki on each LORi, wherein i represents a number, $i\geq1$; an activity image reconstruction module, configured to acquire an activity image based on all coincidence events of all lines of response LORi passing through the target object; a second coincidence event screening module, configured to based on all lines of response LORi passing through the target object, screen lines of response LORj on which coincidence events' number satisfies a preset reference value, wherein j represents a number, $j\geq1$; a pixel value-time difference statistical distribution acquisition module, configured to determine pixel points Pj where the respective, screened lines of response LORj intersect with the activity image, and determine a pixel value-time difference statistical distribution Tj corresponding to each line of response LORj based on the pixel points Pj respectively; a coincidence event-time difference statistical distribution acquisition module, configured to determine a coincidence event-time difference statistical distribution Mj corresponding to each screened line of response LORj; a calculation module, configured to calculate a maximum value Yj of the inner product between the pixel value-time difference statistical distribution Tj and the coincidence event-time difference statistical distribution Mj, and determine a time offset difference value $\delta\triangle j$ corresponding to the maximum value Yj for each line of response LORj; and a time calibration value acquisition module, configured to determine a time calibration value based on the offset difference value $\delta\triangle j$.

According to an embodiment of the disclosure, the sampling data comprises scintillator IP information and time information of pulse signals; and the coincidence event screening module is further configured to determine energy information of the pulse signals based on the sampling data, and determine coincidence events based on the time information and the energy information of the pulse signals, and determine the lines of response LOR corresponding to the coincidence events based on the scintillator IP information.

According to an embodiment of the disclosure, the time calibration device further comprises: a recording module, configured to record time differences of the coincidence events and scintillator IP information of scintillators at two ends of the corresponding line of response LOR.

According to an embodiment of the disclosure, the first coincidence event screening module is configured to based on priori information, determine whether a straight line where the line of response LOR lies has an intersection point with the target object, and determine whether the line of response LOR passes through the target object based on a number of intersection points.

According to an embodiment of the disclosure, the priori information comprises the number of scintillator IPs between the spaced scintillators at two ends of the response line LOR, wherein the number of scintillator IPs between the spaced scintillators is determined based on whether the inner diameters of the target object in various directions intersect with the straight line where the response line LOR lies.

According to an embodiment of the disclosure, the activity image reconstruction module is configured to reconstruct the activity image using filtered back projection or an iterative method.

According to an embodiment of the disclosure, the second coincidence event screening module comprises: a reference value setting module, configured to set a reference value for comparing the number of coincidence events; a determining module, configured to determine whether the number of coincidence events is greater than the reference value; and a storage module, configured to store lines of response LOR on which the coincidence events' number is greater than the reference value.

According to an embodiment of the disclosure,

the pixel value-time difference statistical distribution acquisition module is configured to determine pixel points Pj where the screened lines of response LORj intersect with the activity image based on a ray tracing method.

According to an embodiment of the disclosure, the pixel value-time difference statistical distribution acquisition module comprises: a pixel time difference acquisition module, configured to calculate a time difference from each pixel point Pj to the endpoints of the corresponding line of response LORj, and record the pixel value of each pixel point Pj; a pixel value accumulation module, configured to determine a plurality of equally spaced time difference intervals, and accumulate pixel values of the pixel points Pj whose time differences fall within the respective time difference interval based on the time difference for each pixel point Pj; and the pixel value-time difference statistical distribution acquisition module is configured to determine the pixel value-time difference statistical distribution Tj corresponding to each line of response LORj by taking the time difference interval as a horizontal axis step size and taking the accumulated pixel value of the pixel points falling within each step size as a vertical axis.

According to an embodiment of the disclosure, the coincidence event-time difference statistical distribution acquisition module is configured to: determine a horizontal axis by taking the horizontal axis step size of the pixel value-time difference statistical distribution Tj as a step size, and by taking the accumulated value of coincidence events whose time differences fall within the respective step size as a vertical axis, determine the coincidence event-time difference statistical distribution Mj corresponding to each line of response LORj.

According to an embodiment of the disclosure, the time calibration value acquisition module is further configured to based on the difference value $\delta\triangle j$ of the time offsets, determine the relationship between the time offsets $\delta cj$ and $\delta dj$ of the scintillators cj and dj at two ends of each line of response LORj, where $\delta cj - \delta dj = \delta\triangle j$; and determine the time offsets $\delta cj$ and $\delta dj$ of the scintillators cj and dj at two ends of each line of response LORj by solving a system of equations using a fitting method, the time offsets $\delta cj$ and $\delta dj$ being the time calibration values of the scintillators cj and dj.

According to an embodiment of the disclosure, the time calibration device further comprises a calibration module, configured to perform time calibration on the time information of pulse signals in the sampling data based on the determined time calibration values: subtracting the time calibration values $\delta ci$ and $\delta di$ from the time data tci and tdi corresponding to the scintillators ci and di in the sampling data, respectively.

According to a fifth aspect of the disclosure, a digitalization apparatus is provided, comprising the time calibration device as described above, wherein the digitalization apparatus uses a detector to detect an object to be detected, uses a sampling module to acquire sampling data, uses the time calibration device to calibrate the sampling data and acquire calibrated time data, thereby forming a digital image based on the calibrated time data.

According to a sixth aspect of the disclosure, a computer device is provided, comprising: a memory, a processor, and a computer program stored on the memory and executable on the processor to implement the steps of the time calibration method as described above.

According to a seventh aspect of the disclosure, a computer-readable storage medium is provided, wherein the storage medium stores a computer program, executable on the processor to implement the steps of the time calibration method as described above.

The solution in the disclosure, by acquiring the pixel value-time difference statistical distribution $T_i$ and the coincidence event-time difference statistical distribution $M_i$ corresponding to each line of response $LOR_i$ based on screened, real coincidence events passing through a target object, and calculating the maximum value $Y_i$ of an inner product of each $T_i$ and $M_i$, and acquiring a difference $\delta\triangle i$ of time offsets corresponding to the maximum value for each line of response $LOR_i$, and then acquiring a time calibration value based on $\delta\triangle i$, is applicable to any shell source, has wide applicability, and does not need to perform Gaussian fitting, thus saving time, improving the efficiency of time calibration, and eliminating the need for highly active drugs.

In addition, because in the embodiment of the disclosure the pixel value-time difference statistical distribution $T_j$ and the coincidence event-time difference statistical distribution $M_j$ corresponding to each line of response $LOR_j$ are acquired based on the screened, real coincidence events corresponding to the lines of response $LOR_j$ which pass through the target object and on which a number of coincidence events satisfies a preset condition, it further eliminates random events and scatter events, thus correspondingly further improving the accuracy of the acquired difference $\delta\triangle j$ of time offsets corresponding to each line of response $LOR_j$, thereby further improving the accuracy of time calibration.

The optional features and additional effects of the embodiments are described in part below, while others can be understood by reading the specification.

**Brief Description of Drawings**

The embodiments of the present application will be described in further detail below with reference to the accompanying drawings. The elements shown in the drawings are not necessarily drawn to scale, and similar or identical reference signs in the drawings represent similar or identical elements, wherein:

FIG. 1 shows a schematic diagram of annihilation position deviation according to the prior art;
FIG. 2 shows a flow chart of a time calibration method according to an embodiment of the disclosure;

FIG. 3 shows a flow chart of a time calibration method according to another embodiment of the disclosure;
FIG. 4 shows an exemplary module diagram of a time calibration device according to an embodiment of the disclosure; and
FIG. 5 shows an exemplary module diagram of a time calibration device according to another embodiment of the disclosure.

**Detailed Description**

To clarify the purpose, technical solution, and advantages of the present invention, detailed descriptions are provided below in conjunction with specific embodiments and accompanying drawings. It should be noted that the following illustrative embodiments and explanations of the present invention are provided for the purpose of explaining the present invention, and are not intended to limit the scope of the present invention.

In the disclosure, the terms "comprise" and any variations thereof are intended to be inclusive, i.e., indicating "include but not limited to". Unless otherwise specified, the term "or" indicates "and/or". The term "based on" means "at least partially based on". The terms "an exemplary embodiment" and "an embodiment" mean "at least one exemplary embodiment". The term "another embodiment" means "at least one additional embodiment". The terms "first", "second", or the like may refer to different or identical objects. Other explicit and implicit definitions may also be referred to in the followings.

The specific embodiments of the present application will be described in detail below with reference to the accompanying drawings. It should be noted that the following description is for the purpose of illustration and is not intended to limit the scope of protection of the disclosure.

FIG. 2 is an exemplary flow chart of a time calibration method according to some embodiments of the disclosure. Specifically, as shown in FIG. 2, the time calibration method may comprise the following steps S120, S130, S140, S150, and S160.

Beginning with implementing the method, step S110 may be performed first: sampling the pulse signals acquired based on the target object, to acquire sampling data.

In the embodiments of the disclosure, the target object may comprise a phantom and/or a biological body, and a simulated radioactive source. Specifically, the phantom and/or biological body comprise but are not limited to animal bodies; the simulated radioactive source comprises but is not limited to small cylinders (which diameter may be less than 20mm), line sources, shell sources, or radioactive sources similar to shell sources, etc., and may also be simulated radioactive sources with regular or irregular shapes.

In the embodiments of the disclosure, sampling data is broadly interpreted to comprise detection data acquired from a physical target object and/or generated simulation data.

In the embodiments of the disclosure, the detection data may be detection data obtained by detecting a physical object, and the detection data is acquired by a detection module. The detection module may be a detection module in various medical imaging devices such as PET, CT, and MR, etc. The detection module comprises a detector which comprises scintillators and photoelectric conversion elements.

In an example of the disclosure, the detection module can be a PET detection module, and its detector comprises scintillators and photoelectric conversion elements. The scintillators comprise but are not limited to lutetium yttrium silicate scintillators, which are used to convert gamma rays into visible light, and the photoelectric conversion elements comprise but are not limited to silicon photomultipliers (SiPM), which are used to convert visible light into pulse signals.

In the embodiments of the disclosure, a pulse signal should be understood as any pulse signal capable of being sampled. Its essence is a physical quantity that changes abruptly in a short period of time and then quickly returns to its initial value, and the physical quantity has certain characteristics. In the disclosure, the pulse signal specifically comprises a scintillation pulse, and there are described using the scintillation pulse as an example in multiple embodiments, in which the terms pulse signal and the scintillation pulse may be interchangeably used. For example, in some embodiments, a scintillation pulse usually has a rising edge and a falling edge, and the rising edge and the falling edge may be expressed by a function model. In some embodiments, the pulse signal is a scintillation pulse signal. For example, a scintillation pulse corresponding to a gamma photon usually exhibits a relatively fast rising edge and a relatively slow falling edge. The rising edge may be characterized by a linear function, and the falling edge can be characterized by an exponential function.

In other embodiments, the waveform of the pulse signal may also be represented as a triangular wave, a square wave, a sine wave, a cosine wave, or some other shaped wave, which will not be repeated here.

In the embodiments of the disclosure, the form of the pulse signal is an electrical pulse signal, and its corresponding characteristics may be the voltage and current of the electrical pulse signal.

In the embodiments of the disclosure, sampling of the pulse signal acquired based on a radioactive source is realized by a sampling module. In an example of the disclosure, the sampling module is a multi-voltage threshold (MVT) sampling module, which performs multi-voltage threshold sampling on the pulse signal. Specifically, the multi-voltage threshold sampling module comprises a threshold setting module, a comparison module, a time-to-digital converter module, and an information storage module. The threshold setting module is used to set sampling thresholds; the comparison module is used to compare the pulse signal with the threshold and output a transition signal; the time-to-digital converter module is used to perform time sampling on the transition signal to acquire the time information of the pulse signal, and the information storage module stores the scintillator identification position information of the detection module and the time information of the pulse signal. Sampling the pulse signal by the MVT sampling module is implemented based on an FPGA chip which integrates a DAC, an LVDS, and a TDC. The DAC integrated in the FPGA chip is used as the threshold setting module, the LVDS is used as the comparison module, and the TDC is used as the time conversion module. The DAC presets thresholds for the LVDS, such as voltage thresholds V1, V2, V3, and V4. The input pulse signal is compared with the thresholds V1-V4 in the LVDS, and sampling is performed at the moments t1-t4 corresponding to the rising edge of the transition signal and at the moments t5-t8 corresponding to the falling edge of the transition signal to acquire sampling data including voltage-time pairs (V1, t1), (V2, t2), (V3, t3), (V4, t4), (V4, t5), (V3, t6), (V2, t7), (V1, t8), where t1-t8 are time data.

In the embodiments of the disclosure, the sampling data comprises the scintillator identification position information of the detection module and the time information of the pulse signal. Among them, the time data t1 may characterize the time information of the pulse signal, and the scintillator identification position information of the detection module may be determined based on the time data t1-t4 of the pulse signal.

In the embodiments of the disclosure, the voltage-time pairs (V1, t1), (V2, t2), (V3, t3), (V4, t4), (V4, t5), (V3, t6), (V2, t7), (V1, t8) in the sampling data are subsequently used to fit and reconstruct the pulse waveform, and the energy information of the pulse signal is acquired by integrating based on the reconstructed pulse waveform. Subsequently, coincidence events are screened based on the time information and the energy information of the pulse signal. Based on the LOR of the screened coincidence events and the time information of the pulse signal, the position information of the gamma photon single event may be determined. It can be seen that the accuracy of the time data affects the screening of coincidence events and the accuracy of the position information of the single event, that is, the accuracy of the annihilation position.

In the embodiments of the disclosure, simulation data may be, for example, generated simulation data, which can be obtained, for example, from simulation software or simulation modules. The simulation software or simulation module is, for example, simulation software or a simulation module for simulating medical imaging equipment and its detection instruments. The simulation software or simulation module comprises a simulation detection module, such as a simulation PET detection module. In a specific example, the sampling data may be acquired from simulation software for simulating PET scanning. Here, in some embodiments, the target object may also comprise a simulated target object.

In an example of the disclosure, the target object (such as a shell source or a line source or a cylindrical radioactive source) is placed at the center of the FOV (Field of View), and sampling is performed using the MVT method. The acquisition time is 10 minutes or more to acquire the sampling data.

Step S120: Based on the sampling data of the target object, determining lines of response LORi (i ≥ 1) passing through the target object and coincidence events Ki (i ≥ 1) on each LORi.

In an embodiment, step S120 may specifically comprise:

S121: Determining all coincidence events and the LORs corresponding to each coincidence event based on the sampling data.

In the embodiments of the disclosure, a coincidence event means two single events corresponding to two pulse signals where a time difference (that is, the difference between the time information of two pulse signals) is within a coincidence time window and where energy information is within a coincidence energy window. Specifically, taking the detection of gamma rays using a PET detector as an example, when two gamma photons generated by an annihilation reaction are detected by the scintillators of two detectors, and the time difference between the time information of the two gamma photons is within a preset coincidence time window, and the energy information of the two gamma photons is both within a preset coincidence energy window, then the event of detecting the two gamma photons may be referred to as a coincidence event. The coincidence time window and coincidence energy window in the embodiments of the disclosure may be determined based on priori information.

In some embodiments, after acquiring the sampling data, it may acquire all coincidence events and the LOR corresponding to each coincidence event based on the sampling data. Specifically, the sampling data may be transmitted to a host computer, and the host computer fits and reconstructs pulse waveforms based on the sampling data. By integrating the reconstructed pulse waveforms, the host computer acquires the energy information of the pulse signal corresponding to each single event. Meanwhile, on the host computer, the scintillator identification position information of the detection module, that is, the scintillator IP number (for example, the scintillators are numbered 1-n), and the time information of the pulse signal may be determined based on the time data of the pulse signal.

In the embodiments of the disclosure, a coincidence time window and a coincidence energy window are preset in a coincidence module of the host computer. Based on the time information and the energy information of the pulse signal acquired from the sampling data, coincidence is performed on the single events corresponding to the pulse signals to determine coincidence events. Also, a recording module is set in the host computer, and by means the recording module, the time difference of the coincidence events and the scintillator IP information of the two scintillators are recorded. The line connecting the centers of the surfaces of the two scintillators is the LOR corresponding to the coincidence event.

In an example, the coincidence time window is set to 4 ns and the coincidence energy window is set to 420 keV-1000 keV in the coincidence module of the host computer. If an annihilation reaction generates two gamma photons γ1 and y2, and based on the sampling data, the host computer determines that the time information of the pulse signal corresponding to the single event of γ1 detected by a scintillator G with an IP number of 6 (taking a PET detector with 48 scintillators as an example) is t1, and that the time information of the pulse signal corresponding to the single event of γ2 detected by a scintillator H with an IP number of 30 is t1', then the time difference is Δt=t1-t1'. If Δt≤4 ns, then the time difference between the two single events is within the coincidence time window. On the host computer, the pulse waveforms of the two single events are fitted and reconstructed, and the energy information e1 of the single event of γ1 and the energy information e2 of the single event of y2 are acquired by integration, respectively. If e1 and e2 are both within the range of 420 keV-1000 keV, then the energy information of the two single events is within the coincidence time window. At this time, the single event of γ1 and the single event of y2 are a pair of coincidence events, and the line connecting the centers of the surfaces of the scintillators G and H is the LORGH corresponding to the coincidence event. The recording module of the host computer records the time difference of the coincidence event as Δt = t1- t1', the scintillator IP information 6 and 30 of the scintillators G and H, and the corresponding LOR, that is, the line connecting the centers of the surfaces of G and H.

Similarly, in the embodiments of the disclosure, it may determine all coincidence events and the corresponding LOR based on the sampling data received by the host computer.

In one embodiment of the disclosure, if a plurality of pairs of coincidence events correspond to the same LOR, then the number of coincidence events on the LOR and the time differences of each coincidence event on the LOR are counted. For example, continuing the above example, if there are 20 pairs of coincidence events that all correspond to LOR$_{GH}$, then the time difference information Δt1-Δt20 of the 20 pairs of coincidence events on LOR$_{GH}$ is counted, and the number of coincidence events 20, the time differences Δt1-Δt20 of each coincidence event, the scintillator IP information 6 and 30 of the scintillators G and H, and the corresponding LOR, that is, the line connecting the centers of the surfaces of G and H are recorded in the recording module.

In an example of the disclosure, the host computer acquires the time information and energy information of all pulse signals based on the sampling data. Based on the preset coincidence time window and coincidence energy window, all coincidence events are screened out, and the time difference of all coincidence events, the Scintillator IP information, and the LORs corresponding to all coincidence events are recorded, so as to facilitate subsequent screening of all LORs passing through the target object (such as a simulated radioactive source) and all coincidence events corresponding to each LOR.

S122: Screening all lines of response LORi passing through the target object and coincidence events Ki on each LORi.

In the embodiments of the disclosure, the coincidence events determined in step S121 may comprise true events, random events, and scatter events. Random events and scatter events will affect the accuracy of the subsequently acquired time calibration values and need to be discarded. Those skilled in the art will understand that the LORs corresponding to the true events in the coincidence events all pass through the target object, that is, have an intersection point with the target object, while random events and scatter events do not pass through the target object, that is, have no intersection point with the target object. Therefore, in an example of the disclosure, the coincidence events corresponding to true events may be screened by whether the LOR corresponding to the coincidence event passes through the target object.

In the embodiments of the disclosure, screening all lines of response LORi passing through the target object and coincidence events Ki on each LORi may be realized by determining whether a straight line where the LOR lies has an intersection point with the target object. Specifically, it may comprise the following step:

Based on priori information, using an intersection point determination module to determine whether the straight line where the LOR lies has an intersection point with the target object.

In one embodiment of the disclosure, the priori information may comprise the number of scintillator IPs between the spaced scintillators of the detection module at two ends of the LOR. The number of scintillator IPs between the spaced scintillators is determined based on whether the target object and the straight line where each LOR lies have an intersection point.

In an example of the disclosure, the acquisition of priori information can be based on different target objects in advance. For target objects with regular shapes or irregular shapes, it may pre-count the number of Scintillator IPs between the Scintillator IPs at the two ends of the corresponding LOR when each LOR has an intersection point with the target object, and use this as priori information and create a priori information lookup table to facilitate consultation.

In an example, regarding the acquisition of priori information of target objects with regular shapes, taking a cylindrical simulated radioactive source with a diameter of less than 20 mm as an example of a target object, the distance f between the straight line where the LOR$_{GH}$ lies and the center point of the cylindrical radioactive source is determined by priori information. If the distance f is less than or equal to the radius d of the cylindrical radioactive source, then the straight line where the LOR$_{GH}$ lies has at least one intersection point with the cylindrical radioactive source and passes through the cylindrical radioactive source. If the distance f=d, the straight line where LOR$_{GH}$ lies is externally tangent to the cross-section of the cylindrical radioactive source. If the scintillator IP number of the scintillator G is 6 and the scintillator IP number of the scintillator H is 20, and when f=d, it is statistically acquired that the number of scintillators spaced between the scintillators G and H is 14. Then, when this cylindrical simulated radioactive source is used as the target object, if the number of scintillator IPs between the scintillators at the two ends of a certain LOR is greater than or equal to 14, then the LOR passes through the target object. Meanwhile, the priori information acquired based on this cylindrical simulated radioactive source is that: if the number of scintillator IPs between the scintillators corresponding to the two ends of the LOR is greater than or equal to 14, then the LOR passes through the target object.

In an example, taking a shell source with a regular shape as an example of a target object, it assumes that the diameter of the shell source used is 500 mm. Assuming that the PET detector used comprises 48 scintillators, if the number of scintillator IPs spaced between two scintillator IPs corresponding to the two ends of the LOR is 12, the LOR between the two scintillators passes through the center point of the shell source, and the distance from the center point of the shell source is 0. If there are 9 scintillator IPs between the two scintillators, then the distance between the line connecting the midpoints of the surfaces of the two crystals and the center point of the shell source is 180 mm, and the LOR between the two scintillators passes through the shell source. If there are 6 scintillator IPs between the two scintillators, then the distance between the line connecting the midpoints of the surfaces of the two scintillators and the center point of the shell source is 250 mm, and the LOR between the two scintillators is externally tangent to the shell source. If the number of scintillator IPs between the two scintillators corresponding to the two ends of a certain LOR is less than 6, then the LOR does not pass through the shell source. Meanwhile, the priori information acquired based on the shell source is that: if the number of Scintillator IPs between the scintillators corresponding to the two ends of the LOR is greater than or equal to 6, then the LOR passes through the target object, i.e., the shell source.

In an embodiment of the disclosure, regarding the acquisition of priori information of target objects with irregular shapes, it is necessary to gradually count to determine whether the target object and the straight line where each LOR lies have an intersection point. Still taking a PET detector including 48 scintillators as an example, when the number of scintillator IPs between the scintillator IP numbers of the scintillators M and N corresponding to the two ends of the $LOR_{MN}$ is greater than or equal to 5, the $LOR_{MN}$ has an intersection point with the target object; by contrast, when the number of spaced scintillator IPs is less than 5, the $LOR_{MN}$ does not have an intersection point with the target object; when the number of scintillator IPs between the Scintillator IP numbers of the scintillators S and R corresponding to the two ends of the $LOR_{SR}$ is greater than or equal to 8, the $LOR_{SR}$ has an intersection point with the target object; by contrast, when the number of spaced Scintillator IPs is less than 8, the $LOR_{SR}$ does not have an intersection point with the target object; ... Similarly, for each LOR formed between any two of the 48 scintillators and having an intersection point with the target object, the critical value of the number of scintillator IPs spaced between the scintillator IP numbers at both ends of the LOR is counted and recorded, and the so as to form a priori information lookup table, to facilitate subsequent determination of whether the LOR corresponding to a coincidence event intersects with the target object based on the LOR and priori information, thereby determining whether the LOR of the coincidence event passes through the target object.

In the embodiments of the disclosure, after acquiring the priori information, based on the priori information, the intersection point determination module is used to determine whether the straight line where the LOR lies has an intersection point with the target object. In an example, taking the shell source in the above example as an example of the target object, by checking with the acquired priori information (if the number of scintillator IPs between the scintillators corresponding to the two ends of the LOR is greater than or equal to 6, then the LOR passes through the target object, i.e., the shell source), if there are 5 scintillator IPs between the scintillator IPs of two scintillators corresponding to the two ends of a certain LOR, then the straight line where the LOR lies does not pass through the target object, and the coincidence events on the LOR are all scatter events or random events, and are discarded; by contrast, if there are 7 scintillator IPs between the scintillator IPs of two scintillators corresponding to the two ends of a certain LOR, then the straight line where the LOR lies passes through the target object, and the coincidence events on the LOR are all true events.

In the embodiments of the disclosure, the coincidence events Ki (i ≥ 1) on the LORi (i ≥ 1) that pass through the target object acquired based on the sampling data are real coincidence events. The coincidence events Ki comprise coincidence event information, and the coincidence event information specifically comprises but is not limited to the number of coincidence events on each LOR, the time difference Δt of each coincidence event, the identification position information of the scintillators corresponding to the two ends of the LOR, the corresponding LOR, that is, the line connecting the centers of the surfaces of the two scintillators, and the number of scintillator IPs between the two scintillators.

In some embodiments, after acquiring the LORi (i ≥ 1) passing through the target object and the coincidence events Ki (i ≥ 1) on each LORi based on the sampling data, the method further comprises the following step:

Recording the screened LORs and their corresponding coincidence event information in a recording module.

In an example, the screened LORs and their corresponding coincidence event information recorded in the recording module comprise but are not limited to: the number of coincidence events on each LOR, the time difference Δt of each coincidence event, the identification position information of the scintillators corresponding to the two ends of the LOR, the corresponding LOR, that is, the line connecting the centers of the surfaces of the two scintillators, and the number of scintillator IPs between the two scintillators. The recording in the recording module facilitates checking and facilitates subsequent reconstruction of the activity image.

Step S130: Based on all coincidence events of all lines of response LORi passing through the target object, acquiring an activity image, and determining pixel points Pi where each line of response LORi passing through the target object intersects with the activity image, and determining a pixel value-time difference statistical distribution Ti corresponding to each LORi based on the pixel points Pi respectively.

In an embodiment, step S130 may specifically comprise:

Step S131: Based on all coincidence events of all lines of response LORi passing through the target object, acquiring an activity image.

In some embodiments, after determining the coincidence events passing through the target object in step S120, it may acquire the activity image based on all coincidence events of all LORi passing through the target object. In an example, step S131 comprises: reconstructing the activity image using filtered back projection or an iterative method based on all coincidence events of all lines of response LORi passing through the target object. The specific implementation of filtered back projection (FBP) or iterative method may refer to the prior art which will not be elaborated here.

The pixel value of each pixel point in the activity image acquired in the embodiments of the disclosure is the activity value. The pixel value has a linear proportional relationship with the number of coincidence events (assuming the linear coefficient is k. The value of k is determined by the specific type of PET detector used. The proportional relationship between the pixel value of the activity image and the number of coincidence events is determined by the type of PET detector), and can be used to characterize the number of coincidence events.

S132: Determining the pixel points Pi where the respective lines of response LORi passing through the target object intersect with the activity image.

In the embodiments of the disclosure, after acquiring the activity image, it may determine the pixel points Pi where each line of response LORi passing through the target object intersects with the activity image. In an example, determining the pixel points Pi where each line of response LORi passing through the target object intersects with the activity image specifically comprises: determining the pixel points Pi where the screened LORi intersect with the activity image acquired in step S131 based on a ray tracing method in the prior art. That is, based on the ray tracing method, it may determine each intersection point, that is, the intersected pixel point, of the LOR screened out in step S120 and the activity image acquired in step S131. The specific operation may refer to the prior art which will not be elaborated here.

S133: Based on the pixel points Pi, determining a pixel value-time difference statistical distribution Ti corresponding to each LORi respectively.

In the embodiments of the disclosure, after determining the pixel points Pi, it may acquire the pixel value-time difference statistical distribution Ti corresponding to each LORi based on the pixel points Pi. Optionally, determining the pixel value-time difference statistical distribution Ti corresponding to each LORi based on the pixel points Pi specifically comprises the following steps:

Step S1331: calculating a time difference from each pixel point Pi to the endpoints of the corresponding line of response LORi, and recording the pixel value of each pixel point Pi;

Step S1332: determining a plurality of equally spaced time difference intervals, and accumulating pixel values of the pixel points Pi whose time differences fall within the respective time difference interval based on the time difference for each pixel point Pi; and

Step S1333: determining the pixel value-time difference statistical distribution Ti corresponding to each line of response LORi by taking the time difference interval as a horizontal axis step size and taking the accumulated pixel value of the pixel points falling within each step size as a vertical axis.

S140: Based on the coincidence events Ki on each line of response LORi, determining a coincidence event-time difference statistical distribution Mi corresponding to each line of response LORi.

In the embodiments of the disclosure, after determining the lines of response LORi (i ≥ 1) passing through the target object and the coincidence events Ki (i ≥ 1) on each LORi based on the sampling data in step S120, it may determine a coincidence event-time difference statistical distribution Mi corresponding to each LORi based on the coincidence events Ki on each LORi. In an example, this specifically comprises:

Determining a horizontal axis by taking the horizontal axis step size of the pixel value-time difference statistical distribution Ti as a step size, and by taking the accumulated value of coincidence events whose time differences fall within the respective step size as a vertical axis, determining the coincidence event-time difference statistical distribution Mi corresponding to each line of response LORi.

In the embodiments of the disclosure, the coincidence event-time difference statistical distribution Mi corresponding to each LORi and the corresponding pixel value-time difference statistical distribution Ti have the same horizontal axis step size, and the ranges set for the horizontal axis are also the same, that is, the horizontal axis settings of Ti and Mi are completely the same, so as to facilitate subsequent calculation of the maximum value Yi of the inner product of each Ti and the corresponding Mi.

Step S150: Based on the pixel value-time difference statistical distribution Ti and the coincidence event-time difference statistical distribution Mi corresponding to each LORi, calculating the maximum value Yi of the inner product of each Ti and Mi, and determining a difference value δ△i of the time offsets corresponding to the maximum value for each LORi.

In the embodiments of the disclosure, based on Ti and Mi, calculating the maximum value Yi of the inner product of each Ti and Mi, and determining the difference value δ△i of the time offsets corresponding to the maximum value for each LORi is realized by a calculation module.

In some embodiments, calculating the maximum value Yi of the inner product of each Ti and Mi, and determining the difference value δ△i of the time offsets corresponding to the maximum value for each LORi, comprises:

Using Ti as a reference, shifting Mi, calculating the inner product of Ti and Mi, and recording a shifting amount of Mi when the maximum value Yi of the inner product of Ti and Mi is reached, the shift amount being δ△i.

In the embodiments of the disclosure, the inner product of Ti and Mi is specifically a value obtained by multiplying the values of the vertical coordinates corresponding to the same horizontal coordinate of Ti and Mi and then adding them together. As can be seen from steps S130 to S140, the horizontal coordinate settings of Ti and Mi are completely the same, and the value of the vertical coordinate corresponding to the horizontal coordinate of Ti is the accumulated value of the pixel values corresponding to the time difference interval range indicated by the horizontal coordinate, and the value of the vertical coordinate corresponding to the horizontal coordinate of Mi is the accumulated value of the coincidence events corresponding to the time difference interval range indicated by the horizontal coordinate. That is, the inner product of Ti and Mi is specifically the value obtained by multiplying the accumulated pixel values and the accumulated coincidence events corresponding to each horizontal coordinate and then adding them together.

Those skilled in the art will understand that, assuming that the scintillators corresponding to the two ends of LORi are ci and di, and the time offsets of the scintillators ci and di are represented by $\delta ci$ and $\delta di$, respectively, and the difference between $\delta ci$ and $\delta di$ is represented by $\delta\triangle i$, that is, $\delta ci - \delta di = \delta\triangle i$, and the time difference Ei of the coincidence event on LORi is represented by:

$$tci + \delta ci - (tdi + \delta di) = Ei,$$

where tci and tdi are the time data in the sampling data corresponding to the scintillators ci and di received by the host computer. By rearranging the formula, it may obtain:

$$tci - tdi + (\delta ci - \delta di) = Ei,$$

When $\delta ci = \delta di$, it may be obtained that:

$$tci - tdi = Ei,$$

It may be seen from the above formula that when $\delta ci = \delta di$, that is, $\delta ci - \delta di = \delta\triangle i = 0$, the time difference of the coincidence events on the LORi is independent of $\delta ci$ and $\delta di$, and is only related to the position where the annihilation event occurs. The time differences between the respective two single events corresponding to two gamma photons generated at different annihilation positions on the LORi are different, while the time differences between the respective two single events corresponding to two gamma photons generated at the same annihilation position at different times are identical. When the LORi corresponds to multiple coincidence events, the number of coincidence events corresponding to the identical time difference is determined. Accordingly, the variation trends of vertical coordinates corresponding to the same horizontal coordinates of Mi and Ti are consistent, the peaks of Mi and Ti shall correspond to each other, and the value Yi obtained by multiplying and adding them together shall be maximum.

That is, when the inner product value of Mi and Ti corresponding to a certain LORi is to the maximum, the peaks of Mi and Ti correspond to teach other, and the time offsets $\delta ci$ and $\delta di$ of the two scintillators ci and di corresponding to the two ends of the LORi satisfy $\delta ci - \delta di = \delta\triangle i$ = 0.

By contrast, if the inner product value of Mi and Ti corresponding to a certain LORi does not reach the maximum value Yi, then the peaks of Mi and Ti do not correspond to each other, and $\delta ci - \delta di = \delta\triangle i$, and $\delta\triangle i$ is not 0.

If the inner product value of Mi and Ti corresponding to a certain LORi does not reach the maximum value Yi (that is, the peaks of Mi and Ti do not correspond to each other), it is caused by $\delta ci - \delta di = \delta\triangle i$, and $\delta\triangle i$ is not 0. If the inner product value of Mi and Ti corresponding to a certain LORi reaches the maximum value Yi (at this time, the peaks of Mi and Ti correspond to each other), it is caused by the time offsets $\delta ci$ and $\delta di$ of the two scintillators ci and di corresponding to the two ends of the LORi satisfying $\delta ci - \delta di = \delta\triangle i$ = 0. Accordingly, it may move Mi and Ti relative to each other such that the inner product value of Mi and Ti reaches the maximum value from a non-maximum value. Meanwhile, the amount of relative movement of Mi and Ti is the difference value $\delta\triangle i$ between $\delta ci$ and $\delta di$. Meanwhile, the time offsets $\delta ci$ and $\delta di$ of the two scintillators ci and di corresponding to the two ends of the LORi satisfy $\delta ci - \delta di = \delta\triangle i$.

In the embodiments of the disclosure, assuming that Mi and Ti corresponding to LORi of two scintillators ci and di corresponding to two ends, Mi may be moved using Ti as a comparison reference. Specifically, the relative movement may be realized by the following operations:

First, determine the range of $\delta\triangle$ through priori information. For example, by fitting through long-term sampling, acquire $\delta ci - \delta di = \delta\triangle i$ of LORi, and obtain the range of $\delta\triangle i$. For example, the range of $\delta\triangle i$ is [-5, 5] ns.

Second, t represents the horizontal coordinate of Ti (the horizontal coordinate step size is the time difference interval range), and $c_T$ represents the value of the vertical coordinate of Ti (accumulated pixel value), $Ti(t, c_T)$ is thus obtained. t represents the horizontal coordinate of Mi (the horizontal coordinate step size is the time difference interval range), and $c_M$ represents the value of the vertical coordinate of Mi (accumulated coincidence events), $Mi(t, c_M)$ is thus obtained. The ranges of the horizontal coordinates t of $Mi(t, c_M)$ and $Ti(t, c_T)$ are set to be the same and are both set to be greater than the range of $\delta\triangle$ acquired based on the priori information. Setting a shift step size of $Mi(t, c_M)$, $Mi(t, c_M)$ is shifted by the step size $\delta M$ each time. After n shifts, $Mi(t + n\delta M, c_M)$ is obtained. The vertical coordinates of the shifted $Mi(t + n\delta M, c_M)$ and $Ti(t, c_T)$ corresponding to the same horizontal coordinates are multiplied and then added together to obtain the value $n\delta M$ when the inner product $<Mi(t + n\delta M, c_M), Ti(t, c_T)>$ reaches the maximum value Yi. At this time, the peaks of $M(t + n\delta M, c_M)$ and $T(t, c_T)>$ correspond to the maximum, and Mi and Ti change from peaks that do not correspond to each other to peaks that correspond to each other. The shift $n\delta M$ is the difference value $\delta\triangle i$ between the time offsets $\delta ci$ and $\delta di$ of the

scintillators ci and di at two ends of the LORi corresponding to Mi and Ti, that is, $\delta ci - \delta di = \delta\triangle i = n*\delta M$.

Step S160: determining a time calibration value based on the $\delta\triangle i$.

In the embodiments of the disclosure, after determining $\delta\triangle i$, it may determine the time calibration value based on the $\delta\triangle i$. In an example, determining the time calibration value based on the $\delta\triangle i$ specifically comprises:

Step S161: Based on the $\delta\triangle i$, determining the relationship $\delta ci - \delta di = \delta\triangle i$ between the time offsets $\delta ci$ and $\delta di$ of the scintillators ci and di of the detection module at two ends of each LORi; and

Step S162: determining the time offsets $\delta ci$ and $\delta di$ of the scintillators ci and di at two ends of each line of response LORi by solving a system of equations using a fitting method, the time offsets $\delta ci$ and $\delta di$ being the time calibration values of the scintillators ci and di.

In the embodiments of the disclosure, after determining the time offsets $\delta ci$ and $\delta di$, i.e., the time calibration values, of the scintillators ci and di of the detection module at two ends of each LORi, the method may also comprise the following step:

Step S170: Based on the determined time calibration value, performing time calibration on the sampling data.

In a specific example of the disclosure, performing calibration on the time data corresponding to the scintillators ci and di of the detection module in the sampling data comprises:

subtracting the time calibration values $\delta ci$ and $\delta di$ from the time data tci and tdi corresponding to the scintillators ci and di of the detection module in the sampling data, respectively.

In the embodiments of the disclosure, by acquiring the pixel value-time difference statistical distribution Ti and the coincidence event-time difference statistical distribution Mi corresponding to each LORi based on the screened true coincidence events passing through the target object, calculating the maximum value Yi of the inner product of each Ti and Mi, acquiring the difference value $\delta\triangle i$ of the time offsets corresponding to the maximum value for each LORi, and then acquiring a time calibration value based on the $\delta\triangle i$, the method is applicable to any target object, has wide applicability, does not need to perform Gaussian fitting, thus saving time, improving the efficiency of time calibration, and does not require the use of high-activity drugs, which improves operational safety. In addition, since in the embodiments of the disclosure the pixel value-time difference statistical distribution Ti and the coincidence event-time difference statistical distribution Mi corresponding to each LORi are acquired based on the screened true coincidence events passing through the target object and random events and scatter events are discarded, the accuracy of the ac-

quired difference value $\delta\triangle i$ of the time offsets corresponding to each LORi is improved, thereby improving the accuracy of the time calibration.

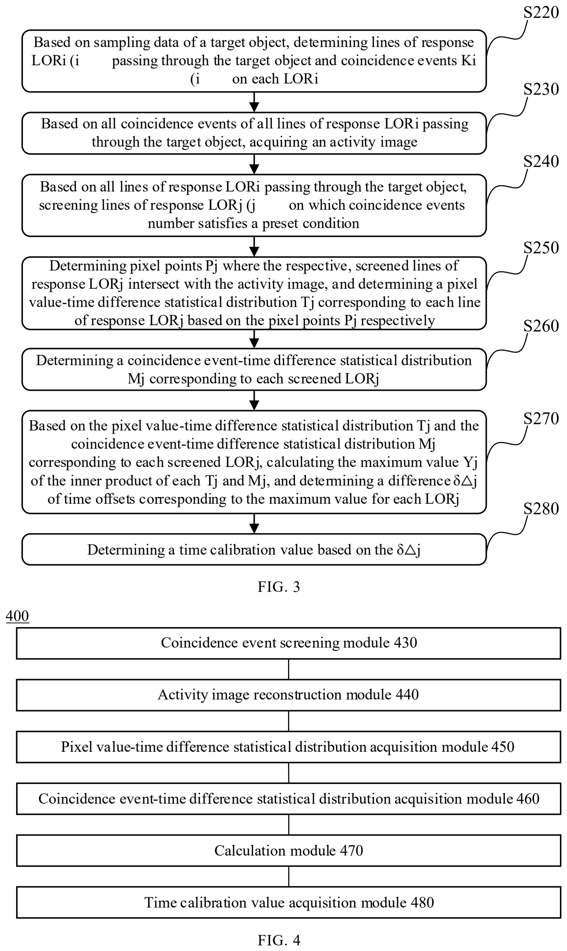

In some embodiments, as an alternative or in addition to the embodiment of FIG. 2, it may screen out lines of response LORj (j≥1) on which coincidence events' number satisfies a preset condition, based on all lines of response LORi passing through the target object. Specifically, the time calibration method shown in the embodiment of FIG. 3 may comprise the following steps S220, S230, S240, S250, S260, S270, and S280.

Beginning with implementing the method, step S210 may be performed first: sampling the pulse signal acquired based on the target object to acquire sampling data.

In the embodiments of the disclosure, for the target object, pulse signals, sampling data and their acquisition, reference may be made to the features in step S110 shown in Figure 2, which will not be elaborated here.

Step S220: Based on sampling data of a target object, determining lines of response LORi (i ≥ 1) passing through the target object and coincidence events Ki (i ≥ 1) on each LORi.

In the embodiments of the disclosure, for determining the lines of response LORi (i ≥ 1) passing through the target object and the coincidence events Ki (i ≥ 1) on each LORi, reference may be made to the features in step S 120 of the time calibration method shown in Figure 2, which will not be elaborated here.

Step S230: Based on all coincidence events of all lines of response LORi passing through the target object, acquiring an activity image.

In the embodiments of the disclosure, for acquiring the activity image, reference may be made to the features in step S131 of the time calibration method shown in Figure 2, which will not be elaborated here.

Step S240: Based on all lines of response LORi passing through the target object, screening lines of response LORj (j ≥ 1) on which coincidence events' number satisfies a preset condition.

Those skilled in the art can understand that if the number of coincidence events corresponding to a certain LOR is too small, the coincidence events on this LOR may usually be considered as scatter events or random events. For example, if there is only one pair of coincidence events on an LOR, the coincidence events on the LOR may be considered as scatter events or random events. In order to further improve the accuracy of the calibration, it is needed to further screen out true events. A certain standard may be set to screen true events from coincidence events to discard scatter events or random events.

In the embodiments of the disclosure, after acquiring all the lines of response LORi passing through the target object, it may screen out the lines of response LORj (j ≥ 1) on which coincidence events' number satisfies a preset condition. In a specific example, step S240 specifically comprises:

Step S241: Setting a reference value for comparing the number of coincidence events.

In the embodiments of the disclosure, the reference value for comparing the number of coincidence events may be set based on priori information, or may be set according to specific needs. For example, the reference value for the number of coincidence events used for comparison may be set to 20. If the number of coincidence events corresponding to each LOR is relatively great, the reference value may be set to a relatively great number; conversely, the reference value can be set smaller.

Step S242: Determining whether the number of coincidence events is greater than the reference value.

In the embodiments of the disclosure, the number of coincidence events corresponding to each LOR is compared with the reference value. If the number of coincidence events corresponding to a certain LOR is greater than the reference value, it meets the requirements, and the LOR is retained and recorded. Otherwise, the LOR is discarded.

Step S243: Storing the LORs on which the coincidence events' number is greater than the reference value using a storage module.

In the embodiments of the disclosure, the LORs that meet the requirements determined in step S242 are stored in the storage module.

Step S250: Determining pixel points $P_j$ where the respective, screened lines of response $LOR_j$ intersect with the activity image, and determining a pixel value-time difference statistical distribution $T_j$ corresponding to each line of response $LOR_j$ based on the pixel points $P_j$ respectively.

In an example, step S250 specifically comprises:

Step S251: Determining pixel points $P_j$ where the respective, screened lines of response $LOR_j$ intersect with the activity image.

In the embodiments of the disclosure, after acquiring the activity image, it may determine the pixel points $P_j$ where the respective, screened lines of response $LOR_j$ passing through the target object intersect with the activity image. In an example, step S251 specifically comprises: determining the pixel points $P_j$ where the respective, screened lines of response $LOR_j$ intersect with the activity image, based on a ray tracing method. The specific operation of the ray tracing method can refer to the prior art, which will not be elaborated here.

Step S252: Based on the pixel points $P_j$, determining a pixel value-time difference statistical distribution $T_j$ corresponding to each $LOR_j$ respectively.

In the embodiments of the disclosure, step S252 specifically comprises:

Calculating a time difference from each pixel point $P_j$ to the endpoints of the corresponding $LOR_j$, and recording the pixel value of each pixel point $P_j$;

Determining a plurality of equally spaced time difference intervals, and based on the time difference of each pixel point $P_j$, counting the accumulated pixel value of the pixel points $P_j$ where the time difference falls within each time difference interval; and

By taking the time difference interval as a horizontal axis step size and taking the accumulated pixel value of the pixel points falling within each step size as a vertical axis, determining the pixel value-time difference statistical distribution $T_j$ corresponding to each $LOR_j$.

Step S260: Determining a coincidence event-time difference statistical distribution $M_j$ corresponding to each screened $LOR_j$.

In an example, step S260 specifically comprises: determining a horizontal axis by taking the horizontal axis step size of the pixel value-time difference statistical distribution $T_j$ as a step size, and by taking the accumulated value of coincidence events whose time differences fall within the respective step size as a vertical axis, determining the coincidence event-time difference statistical distribution $M_j$ corresponding to each line of response $LOR_j$.

In the embodiments of the disclosure, the coincidence event-time difference statistical distribution $M_j$ corresponding to each $LOR_j$ has the same horizontal axis step size as its corresponding pixel value-time difference statistical distribution $T_j$, and their horizontal coordinate range settings are also the same, that is, $T_j$ and $M_j$ have exactly the same horizontal coordinate settings, to facilitate the subsequent calculation of the maximum value $Y_j$ of the inner product between each $T_j$ and its corresponding $M_j$.

Step S270: Based on the pixel value-time difference statistical distribution $T_j$ and the coincidence event-time difference statistical distribution $M_j$ corresponding to each screened $LOR_j$, calculating the maximum value $Y_j$ of the inner product of each $T_j$ and $M_j$, and determining a difference $\delta\triangle j$ of time offsets corresponding to the maximum value for each $LOR_j$.

In some embodiments, calculating the maximum value $Y_j$ of the inner product of each $T_j$ and $M_j$, and determining the difference value $\delta\triangle j$ of the time offsets corresponding to the maximum value for each $LOR_j$, comprises:

Using $T_j$ as a reference, shifting $M_j$, calculating the inner product of $T_j$ and $M_j$, and recording the shifting amount of $M_j$ when the maximum value $Y_j$ of the inner product of $T_j$ and $M_j$ is reached, the shift amount being $\delta\triangle j$.

In the embodiments of the disclosure, calculating the maximum value $Y_j$ of the inner product of each $T_j$ and $M_j$, and determining the difference value $\delta\triangle j$ of the time offsets corresponding to the maximum value for each $LOR_j$, based on the pixel value-time difference statistical distribution $T_j$ and coincidence event-time difference statistical distribution $M_j$ corresponding to each

screened LORj, is realized by a calculation module.

In the embodiments of the disclosure, for calculating the maximum value Yj of the inner product of each Tj and Mj and determining the difference value $\delta\triangle j$ of the time offsets corresponding to the maximum value for each LORj, reference may be made to the features in step S150 of the time calibration method shown in Figure 2, which will not be elaborated here again.

Step S280: Determining a time calibration value based on the maximum value $\delta\triangle j$.

In an example, determining the time calibration value based on the $\delta\triangle j$ specifically comprises:

Step S281: Based on the $\delta\triangle j$, determining the relationship between the time offsets $\delta cj$ and $\delta dj$ of the scintillators cj and dj at two ends of each line of response LORj, where $\delta cj - \delta dj = \delta\triangle j$;

Step S282: determining the time offsets $\delta cj$ and $\delta dj$ of the scintillators cj and dj at two ends of each line of response LORj by solving a system of equations using a fitting method, $\delta cj$ and $\delta dj$ being the time calibration values of the scintillators cj and dj.

In the embodiments of the disclosure, after determining the time offsets $\delta cj$ and $\delta dj$ of the scintillators cj and dj of the detection module at two ends of each LORj, that is, the time calibration values, the following step may also be comprised:

Based on the time offsets $\delta cj$ and $\delta dj$ of the scintillators cj and dj of the detection module, performing calibration on the time data corresponding to the scintillators cj and dj of the detection module in the sampling data, for example: subtracting the time calibration values $\delta cj$ and $\delta dj$ from the time data tcj and tdj corresponding to the scintillators cj and dj of the detection module in the sampling data respectively.

The difference between the embodiment shown in FIG. 3 and the embodiment shown in FIG. 2 is that, by screening the lines of response LORj ($j \geq 1$) on which coincidence events' number satisfies a preset condition based on all LORi passing through the target object, random events and scatter events in the coincidence events are further discarded, screening out as many true events as possible for subsequent obtaining of time calibration values, thereby improving the accuracy of the obtained time calibration values, that is, improving the effect of time calibration.

In some embodiments, the meanings or explanations of identical or similar terms or features in the time calibration method of this embodiment may refer to the time calibration method of the embodiment shown in Figure 2, and the steps and features of the embodiment shown in Figure 2 may be combined in a non-contradictory manner into the time calibration method of this embodiment.

In the embodiments of the disclosure, by acquiring the pixel value-time difference statistical distribution Tj and the coincidence event-time difference statistical distribution Mj corresponding to each LORj based on the screened true coincidence events passing through the target object, calculating the maximum value Yj of the inner product of each Tj and Mj, acquiring the difference value $\delta\triangle j$ of the time offsets corresponding to the maximum value for each LORj, and then acquiring a time calibration value based on the $\delta\triangle j$, the method is applicable to any target object, has wide applicability, does not need to perform Gaussian fitting, thus saving time, improving the efficiency of time calibration, and does not require the use of drugs with high activity, which improves operational safety. In addition, since in the embodiments of the disclosure the pixel value-time difference statistical distribution Tj and the coincidence event-time difference statistical distribution Mj corresponding to each LORj are acquired based on the true coincidence events corresponding to the LORs that pass through the target object and whose coincidence events' number satisfies a preset condition, random events and scatter events are further discarded, the accuracy of the acquired difference value $\delta\triangle j$ of the time offsets corresponding to each LORj is accordingly further improved, thereby further improving the accuracy of the time calibration.

FIG. 4 is an exemplary module diagram of a time calibration device according to some embodiments of the disclosure.

FIG. 4 shows a time calibration device, which may comprise:

a coincidence event screening module 430, an activity image reconstruction module 440, a pixel value-time difference statistical distribution acquisition module 450, a coincidence event-time difference statistical distribution acquisition module 460, a calculation module 470, and a time calibration value acquisition module 480.

In some embodiments, the coincidence event screening module 430 is configured to, based on the sampling data of a target object, determine lines of response LORi ($i \geq 1$) passing through the target object and coincidence events Ki ($i \geq 1$) on each LORi.

In an example, the coincidence event screening module 430 comprises an intersection point determination module for determining whether a straight line where the LOR lies has an intersection point with the target object, and determine whether the LOR passes through the target object based on the number of intersection points. Specifically, the intersection point determination module may determine whether the straight line where the LOR lies has an intersection point with the target object based on priori information. In a specific example, the priori information comprises the number of scintillator IPs between the spaced scintillators of the detection module at two ends of the LOR, and the number of spaced scintillator IPs is determined based on whether the inner diameters of the target object in various directions and the straight line where the LOR lies have an intersection point.

In some embodiments, the activity image reconstruction module 440 is configured to acquire an activity image based on all coincidence events of all lines of response LORi passing through the target object. In an example, the activity image reconstruction module 440 acquires the activity image based on all coincidence events of all lines of response LORi passing through the target object, comprising: reconstructing the activity image using filtered back projection or an iterative method.

In some embodiments, the pixel value-time difference statistical distribution acquisition module 450 is configured to determine pixel points Pi where each line of response LORi passing through the target object intersects with the activity image, and to determine a pixel value-time difference statistical distribution Ti corresponding to each LORi based on the pixel points Pi respectively.

In an example, the pixel value-time difference statistical distribution acquisition module 450 comprises a pixel point acquisition module and a time difference statistical distribution acquisition module. The pixel point acquisition module is used to determine pixel points Pi where the screened LORi intersects with the activity image based on a ray tracing method. The time difference statistical distribution acquisition module is used to determine a pixel value-time difference statistical distribution Ti corresponding to each LORi based on the pixel points Pi respectively.

In a specific example, the time difference statistical distribution acquisition module comprises a pixel point time difference acquisition module, a pixel value accumulation module, and a pixel value-time difference statistical distribution Ti acquisition module. The pixel point time difference acquisition module is used to calculate a time difference from each pixel point Pi to the endpoints of the corresponding LORi, and record the pixel value of each pixel point Pi. The pixel value accumulation module is used to determine a plurality of equally spaced time difference intervals, and based on the time difference of each pixel point Pi, count the accumulated pixel value of the pixel points Pi where the time difference falls within each time difference interval. The pixel value-time difference statistical distribution Ti acquisition module is used to determine the pixel value-time difference statistical distribution Ti corresponding to each LORi by taking the time difference interval as the horizontal axis step size and taking the accumulated pixel value of the pixel points falling within each step size as the vertical axis.

In some embodiments, the coincidence event-time difference statistical distribution acquisition module 460 is configured to determine a coincidence event-time difference statistical distribution Mi corresponding to each LORi.

In a specific example, to determine the coincidence event-time difference statistical distribution Mi corresponding to each LORi, the coincidence event-time difference statistical distribution acquisition module 460 is configured to: determine a horizontal axis by taking the horizontal axis step size of the pixel value-time difference statistical distribution Ti as a step size, and by taking the accumulated value of coincidence events whose time differences fall within the respective step size as a vertical axis, determine the coincidence event-time difference statistical distribution Mi corresponding to each LORi.

In some embodiments, the calculation module 470 is configured to calculate the maximum value Yi of the inner product of each Ti and Mi, and determine a difference $\delta\triangle i$ of time offsets corresponding to the maximum value for each LORi.

In some embodiments, the time calibration value acquisition module 480 is configured to determine time calibration values based on $\delta\triangle i$. In an example, the time calibration values are the time offsets $\delta ci$ and $\delta di$ of the scintillation crystals ci and di of the detection module at two ends of each LORi.

In a specific example, the time calibration value acquisition module 480 comprises a fitting module, which is configured to, based on the $\delta i$, determine the relationship $\delta ci - \delta di = \delta i$ between the time offsets $\delta ci$ and $\delta di$ of the scintillation crystals ci and di of the detection module at two ends of each LORi; and determine the time offsets $\delta ci$ and $\delta di$ of the scintillators ci and di at two ends of each line of response LORi by solving a system of equations using a fitting method.

In some embodiments, the calculation module and the time calibration value acquisition module may be realized by the same device.

In some embodiments, the time calibration device may further comprise a coincidence module, for determining all coincidence events and the LOR corresponding to each coincidence event based on the sampling data. In an example, the sampling data may comprise scintillator IP information of the detection module and time information of the pulse signal. The coincidence module comprises a pulse signal energy information acquisition module, for determining the energy information of the pulse signals based on the sampling data, and determining coincidence events based on the time information and the energy information of the pulse signals, and determining the lines of response LOR corresponding to the coincidence events based on the scintillator IP information.

In some embodiments, the time calibration device may also comprise a recording module, for recording the time difference of the coincidence events and the scintillator number information of the scintillators of the detection module at two ends of the corresponding LOR.

In some embodiments, the time calibration device also comprises a calibration module. The sampling data comprises the scintillator number information of the detection module and the time information of the pulse signals. The calibration module is used to perform time calibration on the time information of pulse signals in the sampling data based on the time calibration value.

In an example, the calibration module performs time calibration on the time information of the pulse signal in the sampling data based on the time calibration value, that is, performing calibration on the time data corresponding to the scintillators $c_i$ and $d_i$ of the detection module in the sampling data, comprising: subtracting the time calibration values $\delta c_i$ and $\delta d_i$ from the time data $t_{ci}$ and $t_{di}$ corresponding to the scintillation crystals $c_i$ and $d_i$ of the detection module, respectively.

In the embodiments of the disclosure, by acquiring the pixel value-time difference statistical distribution $T_i$ and the coincidence event-time difference statistical distribution $M_i$ corresponding to each LORi based on the screened true coincidence events passing through the target object, calculating the maximum value $Y_i$ of the inner product of each $T_i$ and $M_i$, acquiring the difference value $\delta\triangle_i$ of the time offsets corresponding to the maximum value for each LORi, and then acquiring a time calibration value based on the $\delta\triangle_i$, it is applicable to any target object, has wide applicability, and does not need to perform Gaussian fitting, thus saving time and improving the efficiency of time calibration. In addition, since in the embodiments of the disclosure the pixel value-time difference statistical distribution $T_i$ and the coincidence event-time difference statistical distribution $M_i$ corresponding to each LORi are acquired based on the screened true coincidence events passing through the target object and random events and scatter events are discarded, the accuracy of the acquired difference value $\delta\triangle_i$ of the time offsets corresponding to each LORi is improved, thereby improving the accuracy of the time calibration.

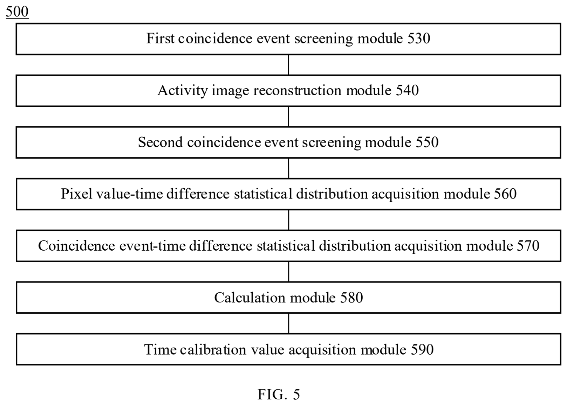

In some embodiments, as an alternative or in addition to the embodiment of FIG. 4, two coincidence event screening modules may be provided, where the first coincidence event screening module corresponds to the coincidence event screening module of the embodiment shown in FIG. 4, and the second coincidence event screening module is configured to, based on all lines of response LORi passing through the target object, screen the lines of response LORj on which the coincidence events' number satisfies a preset reference value. Specifically, as shown in the embodiment of a time calibration device in FIG. 5, the time calibration device may comprise:

a first coincidence event screening module 530, configured to based on sampling data of a target object, determine lines of response LORi ($i \geq 1$) passing through the target object and coincidence events Ki ($i \geq 1$) on each LORi;

an activity image reconstruction module 540, configured to acquire an activity image based on all coincidence events of all lines of response LORi passing through the target object;

a second coincidence event screening module 550, configured to based on all lines of response LORi

passing through the target object, screen lines of response LORj on which coincidence events' number satisfies a preset reference value;

a pixel value-time difference statistical distribution acquisition module 560, configured to determine pixel points Pj where the respective, screened lines of response LORj intersect with the activity image, and based on the pixel points Pj respectively, determine a pixel value-time difference statistical distributions Tj corresponding to each LORj;

a coincidence event-time difference statistical distribution acquisition module 570, configured to determine a coincidence event-time difference statistical distribution Mj corresponding to each screened LORj;

a calculation module 580, configured to calculate a maximum value Yj of the inner product of each Tj and Mj, and determine the difference $\delta\triangle_j$ of time offsets corresponding to the maximum value Yj for each LORj; and

a time calibration value acquisition module 590, configured to determine a time calibration value based on the $\delta\triangle_j$.

The difference between the present embodiment and the embodiment shown in FIG. 4 is that, in the present embodiment there are two coincidence event screening modules, wherein the first coincidence event screening module 530 corresponds to the coincidence event screening module of the embodiment shown in FIG. 4, both being configured to determine lines of response LORi ($i \geq 1$) passing through the target object and coincidence events Ki ($i \geq 1$) on each LORi, specifically with reference to the embodiment shown in FIG. 4. In addition, in the present embodiment it may also comprise a second coincidence event screening module 550, which is configured to screen lines of response LORj on which the coincidence events' number satisfies a preset reference value based on all lines of response LORi passing through the target object. In an example, the second coincidence event screening module comprises: a reference value setting module, for setting a reference value for comparing the number of coincidence events; a determination module, for determining whether the number of coincidence events is greater than the reference value; and a storage module, for storing the LORs on which the number of coincidence events is greater than the reference value.

In the present embodiment, by providing the second coincidence event screening module 550, the lines of response LORj ($j \geq 1$) on which the coincidence events' number satisfies a preset condition are screened out based on all lines of response LORi passing through the target object, further discarding random events and scatter events in the coincidence events, and screening out as many true events as possible for subsequently acquiring time calibration values, thereby improving the accuracy of the obtained time calibration values, that is, improving the effect of time calibration.

In the time calibration device of the embodiments of the disclosure, the components or features of the time calibration method embodiment shown in Figure 4 may be included in a non-contradictory manner, or the embodiment shown in the present figure may be combined with the embodiment shown in Figure 4 to obtain new embodiments, and vice versa.

In the embodiments of the disclosure, by acquiring the pixel value-time difference statistical distribution $T_j$ and the coincidence event-time difference statistical distribution $M_j$ corresponding to each $LOR_j$ based on the screened true coincidence events passing through the target object, calculating the maximum value $Y_j$ of the inner product between each $T_j$ and $M_j$, acquiring the difference value $\delta\triangle_j$ of the time offsets corresponding to the maximum value for each $LOR_j$, and then acquiring a time calibration value based on the $\delta\triangle_j$, it is applicable to any target object, has wide applicability, and does not need to perform Gaussian fitting, thus saving time, improving the efficiency of time calibration, and does not require the use of drugs with high activity, which improves operational safety. In addition, since in the embodiments of the disclosure the pixel value-time difference statistical distribution $T_j$ and the coincidence event-time difference statistical distribution $M_j$ corresponding to each $LOR_j$ are acquired based on the true coincidence events corresponding to the LORs that pass through the target object and whose coincidence events' number satisfies a preset condition, random events and scatter events are further discarded, accordingly further improving the accuracy of the acquired difference value $\delta\triangle_j$ of the time offsets corresponding to each $LOR_j$, thereby further improving the accuracy of the time calibration.

In some embodiments in the disclosure also provided is a digitalization apparatus comprising the time calibration device mentioned in the above embodiments, a detection module and a sampling module. The digitalization apparatus may be used to calibrate time data of pulse signals obtained based on a target object, improving calibration efficiency, calibration effect and calibration safety. The digitalization apparatus uses the time calibration device to detect an object to be detected, acquire sampling data, calibrate the sampling data based on the time calibration values to acquire calibrated time data, and form a digital image based on the acquired coincidence events and calibrated time data. In a specific example, the digitalization apparatus is a Positron Emission Tomography (PET).

In some embodiments, the detection module is configured to acquire pulse signals based on a target object. The detection module 410 comprises a detector which comprises scintillators and a photoelectric conversion element , wherein the scintillators are used to convert gamma rays into visible light, and the photoelectric conversion elements are used to convert visible light into pulse signals.

In some embodiments, the sampling module is configured to sample the pulse signal to acquire sampling data. For the sampling data, reference may be made to the method embodiments, which will not be elaborated here.

In an example, the sampling module is a multi-voltage threshold sampling module which comprises a threshold setting module, a comparison module, a time-to-digital conversion module, and an information storage module, wherein the threshold setting module is used to set sampling thresholds, the comparison module is used to compare the pulse signals with the thresholds and output a transition signals; the time-to-digital conversion module is used to perform time sampling on the transition signal to acquire the time information of the pulse signal, and the information storage module stores the scintillator number information of the detection module and the time information of the pulse signal.

In the embodiments of the disclosure, the digitization apparatus may directly acquire the annihilation position based on the calibrated time data and coincidence events, thereby directly forming a digital image (such as a PET image) without requiring complex image reconstruction methods, simplifying image reconstruction.

In the apparatus described in the embodiments of the disclosure, the features of the methods described in the embodiments of the disclosure may be combined, and vice versa.

In some embodiments in the disclosure also provided is a computer device, comprising: a memory, a processor, and a computer program stored on the memory and executable on the processor, wherein when executed by the processor, the computer program implements the steps of any method described in the embodiments of the disclosure.

Although not shown, in some embodiments, a computer-readable storage medium is also provided, storing a computer program configured to implement the steps of any method described in the embodiments of the disclosure when executed. The computer program contains program modules/units constituting the apparatus according to the embodiments of the disclosure, and when executed, the computer program constituted by the program modules/units can implement functions corresponding to each step described in the methods of the above embodiments. The computer program may also run on electronic devices as described in the embodiments of the disclosure.

This text has described basic concepts, and obviously, for those skilled in the art, the above detailed disclosure serves only as examples and does not constitute limitations on the disclosure. Although not explicitly stated, those skilled in the art may make various modifications, improvements, and corrections to the disclosure. The modifications, improvements, and corrections suggested in the disclosure still fall within the spirit and scope of the exemplary embodiments of the disclosure.

Meanwhile, the disclosure has used specific words to describe the embodiments of the disclosure. "an embodiment", "one embodiment", and/or "some embodiments" refer to a certain feature, structure, or characteristic related to at least one embodiment of the disclosure. Therefore, it should be emphasized and noted that when "an embodiment" or "one embodiment" or "an alternative embodiment" is mentioned twice or more times at different locations in the disclosure, they do not necessarily refer to the same embodiment. Furthermore, certain features, structures, or characteristics in one or more embodiments of the disclosure may be appropriately combined.

Moreover, those skilled in the art can understand that various aspects of the disclosure can be illustrated and described through several patentable types or circumstances, including any new and useful process, machine, product, or composition of matter, or any new and useful improvement thereof. Accordingly, various aspects of the disclosure may be executed entirely by hardware, entirely by software (including firmware, resident software, microcode, etc.), or by a combination of hardware and software. Any such hardware or software may be referred to as "data blocks", "modules", "engines", "units", "components" or "systems". Furthermore, aspects of the disclosure may appear as a computer product located in one or more computer-readable media, containing computer-readable program coding.

The computer storage medium may include a propagation data signal containing computer program coding, for example on a baseband or as part of a carrier. This propagation signal may take various forms, including electromagnetic forms, optical forms, or suitable combinations thereof. The computer storage medium can be any computer-readable medium other than computer-readable storage media that can communicate, propagate, or transmit programs for use through connection to an instruction execution system, apparatus, or device. Program coding located on computer storage media can be transmitted through any suitable medium, including wireless, cable, fiber optic cable, RF, or similar media, or any combination of the above media.

The computer program coding required for various operations of the disclosure may be written in any one or more programming languages, including object-oriented programming languages such as Java, Scala, Smalltalk, Eiffel, JADE, Emerald, C++, C#, VB.NET, Python, etc., conventional procedural programming languages such as C language, Visual Basic, Fortran 2003, Perl, COBOL 2002, PHP, ABAP, dynamic programming languages such as Python, Ruby and Groovy, or other programming languages. The program coding may run entirely on the user's computer, run as a standalone software package on the user's computer, run partly on the user's computer and partly on a remote computer, or run entirely on a remote computer or server. In the latter case, the remote computer may be connected to the user's computer through any form of network, such as a local area network (LAN) or wide area network (WAN), or connected to external computers (for example through the Internet), or in a cloud computing environment, or used as a service such as Software as a Service (SaaS).

Furthermore, unless explicitly stated in the claims, the processing elements and sequences, use of numerical letters, or other names mentioned in the disclosure are not intended to limit the sequence of processes and methods of the disclosure. Although the above disclosure has discussed some currently considered useful embodiments of the invention through various examples, it should be understood that such details are only for illustrative purposes, and the additional claims are not limited to the disclosed embodiments; rather, the claims are intended to cover all modifications and equivalent combinations that fall within the substance and scope of the embodiments of the disclosure. For example, although the system components described above can be implemented through hardware devices, they can also be implemented purely through software solutions, such as installing the described system on existing servers or mobile devices.

Similarly, it should be noted that to simplify the expression of the disclosure and thus help understand one or more embodiments of the invention, in the above description of the embodiments of the disclosure, various features are sometimes consolidated into a single embodiment, drawing, or description thereof. However, this disclosed method does not mean that the disclosure requires more features than those mentioned in the claims. In fact, the features of the embodiments may be fewer than all the features of a single embodiment described above.

In some embodiments where numbers are used to describe components and quantity attributes, it should be understood that such numbers used for describing embodiments are modified by words like "about", "approximately" or "substantially" in some examples. Unless otherwise stated, "about", "approximately" or "substantially" indicates that the stated number allows for $\pm 20\%$ variation. Accordingly, in some embodiments, numerical parameters used in the specification and claims are approximate values, which may vary depending on the characteristics required by individual embodiments. In some embodiments, numerical parameters should consider specified significant figures and use general rounding methods. Although some embodiments of the disclosure use numerical ranges and parameters to confirm their scope breadth as approximate values, in specific implementations, such numbers are set as precisely as possible within feasible ranges.

For each patent, patent application, published patent application, and other materials (such as articles, books, manuals, publications, documents, etc.) cited in this application, the entire contents thereof are hereby incorporated by reference in their entirety, and excluded from incorporation by reference are any application history files that are inconsistent or in conflict with the content of this application, as well as any documents (currently or subsequently added to this application) that limit the broadest scope of the claims of this application. It should be noted that, in the event of any inconsistency or conflict between the descriptions, definitions, and/or usage of terms in the appendices of this application and those set forth herein, the descriptions, definitions, and/or usage of terms provided in this application shall govern.

Finally, it should be understood that the embodiments described in this application are provided merely to illustrate the principles of the present embodiments. Other modifications may also fall within the scope of this application. Therefore, by way of example rather than limitation, alternative configurations of the embodiments may be viewed as consistent with the teachings of this application. Accordingly, the embodiments of this application are not limited solely to the embodiments expressly introduced and described herein.

**Claims**

1. A time calibration method, **characterized by** comprising:

   based on sampling data of a target object, determining lines of response (LORi) passing through the target object and coincidence events Ki on each LORi, wherein i represents a number, $i \geq 1$;
   acquiring an activity image based on all coincidence events of all lines of response LORi passing through the target object, and determining pixel points Pi where the respective LORi passing through the target object intersect with the activity image, and determining a pixel value-time difference statistical distribution Ti corresponding to each LORi based on the pixel points Pi respectively;
   determining a coincidence event-time difference statistical distribution Mi corresponding to each LORi based on the coincidence events Ki on each line of response LORi;
   based on the pixel value-time difference statistical distribution Ti and the coincidence event-time difference statistical distribution Mi, calculating a maximum value Yi of inner product of the pixel value-time difference statistical distribution Ti and the coincidence event-time difference statistical distribution Mi and determining a time offset difference value $\delta \triangle i$ corresponding to the maximum value Yi for each line of response LORi; and

   determining a time calibration value based on the offset difference value $\delta \triangle i$.

2. The time calibration method according to claim 1, **characterized in that** the lines of response LORi passing through the target object comprise: the lines of response LORi having at least one intersection point with the target object, or the lines of response LORi being externally tangent to or intersecting with the target object.

3. The time calibration method according to claim 1, **characterized in that** based on sampling data of a target object, determining lines of response (LORi) passing through the target object and coincidence events Ki on each LORi, comprises:

   determining all coincidence events and lines of response LORs corresponding to each coincidence event based on the sampling data; and screening all lines of response LORi passing through the target object and coincidence events Ki on each LORi.

4. The time calibration method according to claim 3, **characterized in that** screening all lines of response LORi passing through the target object and coincidence events Ki on each LORi, comprises: determining whether a straight line where the line of response LOR lies has an intersection point with the target object based on priori information.

5. The time calibration method according to claim 4, **characterized in that** the priori information comprises the number of scintillator IPs between the spaced scintillators at two ends of the response line LOR, wherein the number of scintillator IPs between the spaced scintillators is determined based on whether the inner diameters of the target object in various directions intersect with the straight line where the response line LOR lies.

6. The time calibration method according to claim 1, **characterized in that** acquiring the activity image based on all coincidence events of all LORi passing through the target object, comprises: reconstructing the activity image using filtered back projection or an iterative method based on all coincidence events on all lines of response LORi passing through the target object.

7. The time calibration method according to claim 1, **characterized in that** determining pixel points Pi where the respective LORi passing through the target object intersect with the activity image, comprises: determining pixel points Pi where the respective, screened lines of response LORi intersect with the activity image based on a raytracing method.

8. The time calibration method according to claim 1, **characterized in that** determining the pixel value-time difference statistical distribution Ti corresponding to each LORi based on the pixel points Pi respectively, comprises:

calculating a time difference from each pixel Pi to the endpoints of the corresponding line of response LORi for each pixel point Pi, and recording the pixel value of each pixel point Pi; determining a plurality of equally spaced time difference intervals, and accumulating pixel values of the pixel points Pi whose time differences fall within the respective time difference interval based on the time difference for each pixel point Pi; and determining the pixel value-time difference statistical distribution Ti corresponding to each line of response LORi by taking the time difference interval as a horizontal axis step size and taking the accumulated pixel value of the pixel points falling within each step size as a vertical axis.

9. The time calibration method according to claim 8, **characterized in that** determining a coincidence event-time difference statistical distribution Mi corresponding to each LORi based on the coincidence events Ki on each line of response LORi, comprises: determining a horizontal axis by taking the horizontal axis step size of the pixel value-time difference statistical distribution Ti as a step size, and by taking the accumulated value of coincidence events whose time differences fall within the respective step size as a vertical axis, determining the coincidence event-time difference statistical distribution Mi corresponding to each line of response LORi.

10. The time calibration method according to claim 1, **characterized in that** calculating the maximum value Yi of inner product of the pixel value-time difference statistical distribution Ti and the coincidence event-time difference statistical distribution Mi and determining the time offset difference value $\delta\triangle i$ corresponding to the maximum value Yi for each line of response LORi, comprises: using the pixel value-time difference statistical distribution Ti as a reference, shifting the coincidence event-time difference statistical distribution Mi, calculating the inner product of the pixel value-time difference statistical distribution Ti and the coincidence event-time difference statistical distribution Mi, and statistically acquiring a shift amount of the coincidence event-time difference statistical distribution Mi when the maximum value Yi of the inner product is reached, the shift amount being the offset difference value $\delta\triangle i$.

11. The time calibration method according to claim 1, **characterized in that** determining a time calibration value based on the offset difference value $\delta\triangle i$, comprises:

based on the time offset difference value $\delta\triangle i$, determining the relationship between the time offsets $\delta ci$ and $\delta di$ of the scintillators ci and di at two ends of each response line LORi, where $\delta ci - \delta di = \delta\triangle i$; and determining the time offsets $\delta ci$ and $\delta di$ of the scintillators ci and di at two ends of each line of response LORi by solving a system of equations using a fitting method, the time offsets $\delta ci$ and $\delta di$ being the time calibration values of the scintillators ci and di.

12. The time calibration method according to claim 11, further comprising: performing time calibration on the sampling data based on the determined time calibration values, that is, performing calibration on time data corresponding to the scintillators ci and di in the sampling data based on the time offsets $\delta ci$ and $\delta di$ of scintillators ci and di.

13. The time calibration method according to claim 12, **characterized in that** performing time calibration on the sampling data based on the determined time calibration values, comprises: subtracting the time calibration values $\delta ci$ and $\delta di$ from the time data tci and tdi corresponding to the scintillators ci and di in the sampling data, respectively.

14. A time calibration method, comprising:

based on sampling data of a target object, determining lines of response LORi passing through the target object and coincidence events Ki on each LORi, wherein i represents a number, i≥1; acquiring an activity image based on all coincidence events of all lines of response LORi passing through the target object; based on all lines of response LORi passing through the target object, screening lines of response LORj on which coincidence events' number satisfies a preset condition, wherein j represents a number, j≥1; determining pixel points Pj where the respective, screened lines of response LORj intersect with the activity image, and determining a pixel value-time difference statistical distribution Tj corresponding to each line of response LORj based on the pixel points Pj respectively; determining a coincidence event-time difference statistical distribution Mj corresponding to each screened line of response LORj;

based on the pixel value-time difference statistical distribution Tj and the coincidence event-time difference statistical distribution Mj corresponding to each screened line of response LORj, calculating a maximum value Yj of the inner product of the pixel value-time difference statistical distribution Tj and the coincidence event-time difference statistical distribution Mj, and determining a time offset difference value δ△j corresponding to the maximum value Yj for each line of response LORj; and
determining a time calibration value based on the offset difference value δ△j.

15. The time calibration method according to claim 14, **characterized in that** the lines of response LORi passing through the target object comprise: the lines of response LORi having at least one intersection point with the target object, or the lines of response LORi being externally tangent to or intersecting with the target object.

16. The time calibration method according to claim 14, **characterized in that** based on sampling data, determining lines of response LORi passing through the target object and coincidence events Ki on each LORi, comprises:

   determining all coincidence events and lines of response LOR corresponding to each coincidence event based on the sampling data; and
   screening all lines of response LORi passing through the target object and coincidence events Ki on each LORi.

17. The time calibration method according to claim 16, **characterized in that** screening all lines of response LORi passing through the target object and coincidence events Ki on each LORi, comprises:
determining whether a straight line where the line of response LOR lies has an intersection point with the target object based on priori information.

18. The time calibration method according to claim 17, **characterized in that** the priori information comprises the number of scintillator IPs between the spaced scintillators at two ends of the response line LOR, wherein the number of scintillator IPs between the spaced scintillators is determined based on whether the inner diameters of the target object in various directions intersect with the straight line where the response line LOR lies.

19. The time calibration method according to claim 14, **characterized in that** acquiring the activity image based on all coincidence events of all LORi passing through the target object, comprises:
reconstructing the activity image using filtered back projection or an iterative method based on all coincidence events on all lines of response LORi passing through the target object.

20. The time calibration method according to claim 14, **characterized in that** based on all lines of response LORi passing through the target object, screening lines of response LORj on which coincidence events' number satisfies a preset condition, comprises:

   setting a reference value for comparing the number of coincidence events;
   determining whether the number of coincidence events is greater than the reference value; and
   storing lines of response LORj on which the coincidence events' number is greater than the reference value.

21. The time calibration method according to claim 14, **characterized in that** determining pixel points Pj where the respective, screened lines of response LORj intersect with the activity image, comprises:
determining pixel points Pj where the respective lines of response LORj intersect with the activity image based on a raytracing method.

22. The time calibration method according to claim 14, **characterized in that** determining a pixel value-time difference statistical distribution Tj corresponding to each line of response LORj based on the pixel points Pj respectively, comprises:

   calculating a time difference from each pixel point Pj to the endpoints of the corresponding line of response LORj for each pixel point Pj, and recording the pixel value of each pixel point Pj;
   determining a plurality of equally spaced time difference intervals, and accumulating pixel values of the pixel points Pj whose time differences fall within the respective time difference interval based on the time difference for each pixel point Pj; and
   determining the pixel value-time difference statistical distribution Tj corresponding to each line of response LORj by taking the time difference interval as a horizontal axis step size and taking the accumulated pixel value of the pixel points falling within each step size as a vertical axis.

23. The time calibration method according to claim 22, **characterized in that** determining the coincidence event-time difference statistical distribution Mj corresponding to each screened line of response LORj, comprises:
determining a horizontal axis by taking the horizontal axis step size of the pixel value-time difference statistical distribution Tj as a step size, and by taking the accumulated value of coincidence events whose time differences fall within the respective step size as a vertical axis, determining the coincidence event-time difference statistical distribution Mj corresponding to each line of response LORj.

24. The time calibration method according to claim 14, **characterized in that** calculating a maximum value Yj of the inner product between the pixel value-time difference statistical distribution Tj and the coincidence event-time difference statistical distribution Mj, and determining a time offset difference value $\delta\triangle j$ corresponding to the maximum value for each line of response LORj, comprises:
using the pixel value-time difference statistical distribution Tj as a reference, shifting the coincidence event-time difference statistical distribution Mj, calculating the inner product of the pixel value-time difference statistical distribution Tj and the coincidence event-time difference statistical distribution Mj, and statistically acquiring a shift amount of the coincidence event-time difference statistical distribution Mj when the maximum value Yj of the inner product is reached, the shift amount being the offset difference value $\delta\triangle j$.

25. The time calibration method according to claim 14, **characterized in that** determining a time calibration value based on the offset difference value $\delta\triangle j$, comprises:

based on the offset difference value $\delta\triangle j$, determining the relationship between the time offsets $\delta cj$ and $\delta dj$ of the scintillators cj and dj at two ends of each line of response LORj, where $\delta cj - \delta dj = \delta\triangle j$; and
determining the time offsets $\delta cj$ and $\delta dj$ of the scintillators cj and dj at two ends of each line of response LORj by solving a system of equations using a fitting method, the time offsets $\delta cj$ and $\delta dj$ being the time calibration values of the scintillators cj and dj.

26. The time calibration method according to claim 14, **characterized by** further comprising:
performing time calibration on the sampling data based on the determined time calibration values, that is, performing calibration on time data corresponding to the scintillators cj and dj in the sampling data based on the time offsets $\delta cj$ and $\delta dj$ of scintillators cj and dj.

27. The time calibration method according to claim 26, **characterized in that** performing time calibration on the sampling data based on the determined time calibration values, comprises: subtracting the time calibration values $\delta cj$ and $\delta dj$ from the time data tcj and tdj corresponding to the scintillators cj and dj in the sampling data, respectively.

28. A time calibration device, **characterized by** comprising:

a coincidence event screening module, configured to based on sampling data of a target object, determine lines of response LORi passing through the target object and coincidence events Ki on each LORi, wherein i represents a number, i≥1;
an activity image reconstruction module, configured to acquire an activity image based on all coincidence events of all lines of response LORi passing through the target object;
a pixel value-time difference statistical distribution acquisition module, configured to determine pixel points Pi where the respective lines of response LORi passing through the target object intersect with the activity image, and determine a pixel value-time difference statistical distribution Ti corresponding to each LORi based on the pixel points Pi respectively;
a coincidence event-time difference statistical distribution acquisition module, configured to determine a coincidence event-time difference statistical distribution Mi corresponding to each line of response LORi;
a calculation module, configured to calculate a maximum value Yi of inner product of the pixel value-time difference statistical distribution Ti and the coincidence event-time difference statistical distribution Mi and determine a time offset difference value $\delta\triangle i$ corresponding to the maximum value Yi for each line of response LORi; and
a time calibration value acquisition module, configured to determine a time calibration value based on the offset difference value $\delta\triangle i$.

29. The time calibration device according to claim 28, **characterized in that** the sampling data comprises scintillator IP information and time information of pulse signals;
the coincidence event screening module is further configured to based on the sampling data, determine energy information of the pulse signals, and determine coincidence events based on the time information and the energy information of the pulse signals, and determine the lines of response LOR corresponding to the coincidence events based on the scintillator IP information.

30. The time calibration device according to claim 29, **characterized by** further comprising:
a recording module, configured to record time differences of the coincidence events and scintillator IP information of scintillators at two ends of the corresponding line of response LOR.

31. The time calibration device according to claim 28, **characterized in that** the coincidence event screening module is further configured to based on priori information, determine whether a straight line where the line of response LOR lies has an intersection point with the target object, and determine whether the line of response LOR passes through the target object based on a number of intersection points.

32. The time calibration device according to claim 31, **characterized in that** the priori information comprises the number of scintillator IPs between the spaced scintillators at two ends of the response line LOR, wherein the number of scintillator IPs between the spaced scintillators is determined based on whether the inner diameters of the target object in various directions intersect with the straight line where the response line LOR lies.

33. The time calibration device according to claim 28, **characterized in that** the activity image reconstruction module is configured to reconstruct the activity image using filtered back projection or an iterative method.

34. The time calibration device according to claim 28, **characterized in that** the pixel value-time difference statistical distribution acquisition module is configured to determine pixel points Pi where the screened lines of response LORi intersect with the activity image based on a ray tracing method.

35. The time calibration device according to claim 34, **characterized in that** the pixel value-time difference statistical distribution acquisition module comprises:

a pixel time difference acquisition module, configured to calculate a time difference from each pixel point Pi to the endpoints of the corresponding line of response LORi, and record the pixel value of each pixel point Pi; and
a pixel value accumulation module, configured to determine a plurality of equally spaced time difference intervals, and accumulate pixel values of the pixel points Pi whose time differences fall within the respective time difference interval based on the time difference for each pixel point Pi;
the pixel value-time difference statistical distribution acquisition module is configured to determine the pixel value-time difference statistical distribution Ti corresponding to each line of response LORi by taking the time difference interval as a horizontal axis step size and taking the accumulated pixel value of the pixel points falling within each step size as a vertical axis.

36. The time calibration device according to claim 35, **characterized in that** the coincidence event-time difference statistical distribution acquisition module is configured to:
determine a horizontal axis by taking the horizontal axis step size of the pixel value-time difference statistical distribution Ti as a step size, and by taking the accumulated value of coincidence events whose time differences fall within the respective step size as a vertical axis, determine the coincidence event-time difference statistical distribution Mi corresponding to each line of response LORi.

37. The time calibration device according to claim 28, **characterized in that** the time calibration value acquisition module is further configured to based on the time offset difference value $\delta\triangle i$, determine the relationship between the time offsets $\delta ci$ and $\delta di$ of the scintillators ci and di at two ends of each line of response LORi, where $\delta ci - \delta di = \delta\triangle i$; and determine the time offsets $\delta ci$ and $\delta di$ of the scintillators ci and di at two ends of each line of response LORi by solving a system of equations using a fitting method, the time offsets $\delta ci$ and $\delta di$ being the time calibration values of the scintillators ci and di.

38. The time calibration device according to claim 28, **characterized by** further comprising a calibration module, configured to perform time calibration on the time information of pulse signals in the sampling data based on the determined time calibration values:
subtracting the time calibration values $\delta ci$ and $\delta di$ from the time data tci and tdi corresponding to the scintillators ci and di in the sampling data, respectively.

39. A time calibration device, **characterized by** comprising:

a first coincidence event screening module, configured to based on sampling data of a target object, determine lines of response LORi passing through the target object and coincidence events Ki on each LORi, wherein i represents a number, i≥1;
an activity image reconstruction module, configured to acquire an activity image based on all coincidence events of all lines of response LORi passing through the target object;
a second coincidence event screening module, configured to based on all lines of response LORi passing through the target object, screen lines of response LORj on which coincidence events' number satisfies a preset reference value, wherein j represents a number, j≥1;
a pixel value-time difference statistical distribution acquisition module, configured to determine pixel points Pj where the respective, screened lines of response LORj intersect with the activity image, and determine a pixel value-time difference statistical distribution Tj corresponding to each line of response LORj based on the pixel points Pj, respectively;

a coincidence event-time difference statistical distribution acquisition module, configured to determine a coincidence event-time difference statistical distribution Mj corresponding to each screened line of response LORj;

a calculation module, configured to calculate a maximum value Yj of the inner product between the pixel value-time difference statistical distribution Tj and the coincidence event-time difference statistical distribution Mj, and determine a time offset difference value $\delta\triangle j$ corresponding to the maximum value Yj for each line of response LORj; and

a time calibration value acquisition module, configured to determine a time calibration value based on the offset difference value $\delta\triangle j$.

40. The time calibration device according to claim 39, **characterized in that** the sampling data comprises scintillator IP information and time information of pulse signals; and

the coincidence event screening module is further configured to determine energy information of the pulse signals based on the sampling data, and determine coincidence events based on the time information and the energy information of the pulse signals, and determine the lines of response LOR corresponding to the coincidence events based on the scintillator IP information.

41. The time calibration device according to claim 40, **characterized by** further comprising:

a recording module, configured to record time differences of the coincidence events and scintillator IP information of scintillators at two ends of the corresponding line of response LOR.

42. The time calibration device according to claim 39, **characterized in that** the first coincidence event screening module is configured to based on priori information, determine whether a straight line where the line of response LOR lies has an intersection point with the target object, and determine whether the line of response LOR passes through the target object based on a number of intersection points.

43. The time calibration device according to claim 42, **characterized in that** the priori information comprises the number of scintillator IPs between the spaced scintillators at two ends of the response line LOR, wherein the number of scintillator IPs between the spaced scintillators is determined based on whether the inner diameters of the target object in various directions intersect with the straight line where the response line LOR lies.

44. The time calibration device according to claim 39, **characterized in that** the activity image reconstruction module is configured to reconstruct the activity image using filtered back projection or an iterative method.

45. The time calibration device according to claim 39, **characterized in that** the second coincidence event screening module comprises:

a reference value setting module, configured to set a reference value for comparing the number of coincidence events;

a determining module, configured to determine whether the number of coincidence events is greater than the reference value; and

a storage module, configured to store lines of response LOR on which the coincidence events' number is greater than the reference value.

46. The time calibration device according to claim 39, **characterized in that** the pixel value-time difference statistical distribution acquisition module is configured to determine pixel points Pj where the screened lines of response LORj intersect with the activity image based on a raytracing method.

47. The time calibration device according to claim 46, **characterized in that** the pixel value-time difference statistical distribution acquisition module comprises:

a pixel time difference acquisition module, configured to calculate a time difference from each pixel point Pj to the endpoints of the corresponding line of response LORj, and record the pixel value of each pixel point Pj; and

a pixel value accumulation module, configured to determine a plurality of equally spaced time difference intervals, and accumulate pixel values of the pixel points Pj whose time differences fall within the respective time difference interval based on the time difference for each pixel point Pj;

the pixel value-time difference statistical distribution acquisition module is configured to determine the pixel value-time difference statistical distribution Tj corresponding to each line of response LORj by taking the time difference interval as a horizontal axis step size and taking the accumulated pixel value of the pixel points falling within each step size as a vertical axis.

48. The time calibration device according to claim 39, **characterized in that** the coincidence event-time difference statistical distribution acquisition module is configured to:

determine a horizontal axis by taking the horizontal axis step size of the pixel value-time difference statistical distribution Tj as a step size, and by taking the accumulated value of coincidence events whose time differences fall within the respective step size as a vertical axis, determine the coincidence event-time difference statistical distribution Mj corresponding to each line of response LORj.

49. The time calibration device according to claim 39, **characterized in that** the time calibration value acquisition module is further configured to based on the time offset difference value $\delta\triangle j$, determine the relationship between the time offsets $\delta cj$ and $\delta dj$ of the scintillators cj and dj at two ends of each line of response LORj, where $\delta cj - \delta dj = \delta\triangle j$; and determine the time offsets $\delta cj$ and $\delta dj$ of the scintillators cj and dj at two ends of each line of response LORj by solving a system of equations using a fitting method, the time offsets $\delta cj$ and $\delta dj$ being the time calibration values of the scintillators cj and dj.

50. The time calibration device according to claim 39, **characterized by** further comprising a calibration module, configured to perform time calibration on the time information of pulse signals in the sampling data based on the determined time calibration values: subtracting the time calibration values $\delta ci$ and $\delta di$ from the time data tci and tdi corresponding to the scintillators ci and di in the sampling data, respectively.

51. A digitalization apparatus, **characterized by** comprising the time calibration device according to any one of claims 28 to 50, wherein the digitalization apparatus uses a detector to detect an object to be detected, uses a sampling module to acquire sampling data, uses the time calibration device to calibrate the sampling data and acquire calibrated time data, thereby forming a digital image based on the calibrated time data.

52. A computer device, **characterized by** comprising: a memory, a processor, and a computer program stored on the memory and executable on the processor to implement the steps of the time calibration method according to any one of claims 1 to 27.

53. A computer-readable storage medium, **characterized in that** the storage medium stores a computer program, executable on the processor to implement the steps of the time calibration method according to any one of claims 1 to 27.

FIG. 1

S120

Based on the sampling data of the target object, determining lines of response LORi (i    passing through the target object and coincidence events Ki (i    on each LORi

S130

Based on all coincidence events of all lines of response LORi passing through the target object, acquiring an activity image, and determining pixel points Pi where each line of response LORi passing through the target object intersects with the activity image, and determining a pixel value-time difference statistical distribution Ti corresponding to each LORi based on the pixel points Pi respectively

S140

Based on the coincidence events Ki on each line of response LORi, determining a coincidence event-time difference statistical distribution Mi corresponding to each line of response LORi

S150

Based on the pixel value-time difference statistical distribution Ti and the coincidence event-time difference statistical distribution Mi corresponding to each LORi, calculating the maximum value Yi of the inner product of each Ti and Mi, and determining a difference value $\delta \triangle i$ of the time offsets corresponding to the maximum value for each LORi

S160

Determining a time calibration value based on the $\delta \triangle i$

FIG. 2

Based on sampling data of a target object, determining lines of response LORi (i⩾ passing through the target object and coincidence events Ki (i⩾ on each LORi

S220

Based on all coincidence events of all lines of response LORi passing through the target object, acquiring an activity image

S230

Based on all lines of response LORi passing through the target object, screening lines of response LORj (j⩾ on which coincidence events number satisfies a preset condition

S240

Determining pixel points Pj where the respective, screened lines of response LORj intersect with the activity image, and determining a pixel value-time difference statistical distribution Tj corresponding to each line of response LORj based on the pixel points Pj respectively

S250

Determining a coincidence event-time difference statistical distribution Mj corresponding to each screened LORj

S260

Based on the pixel value-time difference statistical distribution Tj and the coincidence event-time difference statistical distribution Mj corresponding to each screened LORj, calculating the maximum value Yj of the inner product of each Tj and Mj, and determining a difference δ△j of time offsets corresponding to the maximum value for each LORj

S270

Determining a time calibration value based on the δ△j

S280

FIG. 3

400

Coincidence event screening module 430

Activity image reconstruction module 440

Pixel value-time difference statistical distribution acquisition module 450

Coincidence event-time difference statistical distribution acquisition module 460

Calculation module 470

Time calibration value acquisition module 480

FIG. 4

500

| First coincidence event screening module 530 |
|---|

| Activity image reconstruction module 540 |
|---|

| Second coincidence event screening module 550 |
|---|

| Pixel value-time difference statistical distribution acquisition module 560 |
|---|

| Coincidence event-time difference statistical distribution acquisition module 570 |
|---|

| Calculation module 580 |
|---|

| Time calibration value acquisition module 590 |
|---|

FIG. 5

## INTERNATIONAL SEARCH REPORT

| | International application No. |
|---|---|
| | **PCT/CN2023/142100** |

**A.     CLASSIFICATION OF SUBJECT MATTER**

A61B6/03(2006.01)i;  G06T11/00(2006.01)i;  A61B6/00(2024.01)i;  A61B5/055(2006.01)i

According to International Patent Classification (IPC) or to both national classification and IPC

**B.     FIELDS SEARCHED**

Minimum documentation searched (classification system followed by classification symbols)

    IPC: A61B6/-;  A61B5/-;  G06T11/-

Documentation searched other than minimum documentation to the extent that such documents are included in the fields searched

Electronic data base consulted during the international search (name of data base and, where practicable, search terms used)

    CNKI, ENTXT, CNTXT, EPODOC: 锐世, 房磊, 张博, 陈维操, 杨玲莉, 胡云, 时间, 补偿, 校正, 校准, 对准, 精确, 配准, 正电子, 湮灭, 符合, 统计, 分布, 活度, 图像, 响应线, 时间差, 飞行时间, TOF, PET, RUISHI, time, compensate, correct, calibrate, align, precision, register, positron, coincidence, statistics, distribute, activity, image, line, response

**C.     DOCUMENTS CONSIDERED TO BE RELEVANT**

| Category* | Citation of document, with indication, where appropriate, of the relevant passages | Relevant to claim No. |
|---|---|---|
| PX | CN 116019473 A (HEFEI RUISHI DIGITAL TECHNOLOGY CO., LTD.) 28 April 2023 (2023-04-28)<br>    claims 1-53 | 1-53 |
| A | CN 112932517 A (MINFOUND MEDICAL SYSTEMS CO., LTD.) 11 June 2021 (2021-06-11)<br>    description, paragraphs [0002]-[0080], and figures 1-5 | 1-53 |
| A | CN 112998737 A (ZHONGPAI S & T (SHENZHEN) CO., LTD. et al.) 22 June 2021 (2021-06-22)<br>    entire document | 1-53 |
| A | CN 108338805 A (RAYCAN TECHNOLOGY CO., LTD. (SUZHOU)) 31 July 2018 (2018-07-31)<br>    entire document | 1-53 |
| A | KR 20200086814 A (INJE UNIVERSITY INDUSTRY-ACADEMIC COOPERATION FOUNDATION) 20 July 2020 (2020-07-20)<br>    entire document | 1-53 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **12 April 2024** | **17 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/142100** |

**C.      DOCUMENTS CONSIDERED TO BE RELEVANT**

| Category* | Citation of document, with indication, where appropriate, of the relevant passages | Relevant to claim No. |
| --- | --- | --- |
| A | CN 106353786 A (SHANGHAI UNITED IMAGING HEALTHCARE CO., LTD.) 25 January 2017 (2017-01-25)<br>      entire document | 1-53 |

Form PCT/ISA/210 (second sheet) (July 2022)

## INTERNATIONAL SEARCH REPORT
### Information on patent family members

International application No.

**PCT/CN2023/142100**

| Patent document cited in search report | | | Publication date (day/month/year) | Patent family member(s) | | | Publication date (day/month/year) |
|---|---|---|---|---|---|---|---|
| CN | 116019473 | A | 28 April 2023 | None | | | |
| CN | 112932517 | A | 11 June 2021 | None | | | |
| CN | 112998737 | A | 22 June 2021 | None | | | |
| CN | 108338805 | A | 31 July 2018 | JP | 2020503532 | A | 30 January 2020 |
| | | | | JP | 6801906 | B2 | 16 December 2020 |
| | | | | US | 2021356610 | A1 | 18 November 2021 |
| | | | | US | 11280921 | B2 | 22 March 2022 |
| | | | | EP | 3549526 | A1 | 09 October 2019 |
| | | | | EP | 3549526 | B1 | 18 May 2022 |
| | | | | WO | 2018133412 | A1 | 26 July 2018 |
| KR | 20200086814 | A | 20 July 2020 | KR | 102283454 | B1 | 28 July 2021 |
| CN | 106353786 | A | 25 January 2017 | US | 2019235098 | A1 | 01 August 2019 |
| | | | | US | 10598803 | B2 | 24 March 2020 |
| | | | | US | 2018313965 | A1 | 01 November 2018 |
| | | | | US | 10254420 | B2 | 09 April 2019 |
| | | | | WO | 2018059459 | A1 | 05 April 2018 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211736267